(19) Europäisches Patentamt
European Patent Office
Office européen des brevets

(11) EP 1 517 942 B1

(12) # EUROPÄISCHE PATENTSCHRIFT

(45) Veröffentlichungstag und Bekanntmachung des Hinweises auf die Patenterteilung:
**03.05.2006 Patentblatt 2006/18**

(21) Anmeldenummer: **03740207.0**

(22) Anmeldetag: **10.06.2003**

(51) Int Cl.:
***C08G 65/332*** *(2006.01)* ***C07C 69/54*** *(2006.01)*
***C07C 67/08*** *(2006.01)* ***A61L 15/60*** *(2006.01)*

(86) Internationale Anmeldenummer:
**PCT/EP2003/006028**

(87) Internationale Veröffentlichungsnummer:
**WO 2003/104301 (18.12.2003 Gazette 2003/51)**

(54) **(METH)ACRYLESTER VON POLYALKOXYLIERTEM GLYCERIN**
(METH)ACRYLIC ESTERS OF POLYALKOXYLATED GLYCERINE
(METH)ACRYLESTERS DE GLYCERINE POLYALCOXY

(84) Benannte Vertragsstaaten:
**AT BE BG CH CY CZ DE DK EE ES FI FR GB GR HU IE IT LI LU MC NL PT RO SE SI SK TR**

(30) Priorität: **11.06.2002 DE 10225943**
**29.04.2003 DE 10319462**

(43) Veröffentlichungstag der Anmeldung:
**30.03.2005 Patentblatt 2005/13**

(73) Patentinhaber: **BASF Aktiengesellschaft**
**67056 Ludwigshafen (DE)**

(72) Erfinder:
- **POPP, Andreas**
**67134 Birkenheide (DE)**
- **DANIEL, Thomas**
**67165 Waldsee (DE)**
- **SCHRÖDER, Jürgen**
**67071 Ludwigshafen (DE)**
- **JAWOREK, Thomas**
**67169 Kallstadt (DE)**
- **FUNK, Rüdiger**
**65527 Niedernhausen (DE)**
- **SCHWALM, Reinhold**
**67157 Wachenheim (DE)**
- **WEISMANTEL, Matthias**
**63637 Jossgrund-Oberndorf (DE)**
- **RIEGEL, Ulrich**
**66849 Landstuhl (DE)**

(56) Entgegenhaltungen:
**EP-A- 0 777 287**
**WO-A-00/44734**
**DE-A- 10 054 085**
**US-A- 5 482 649**
**US-A- 5 837 789**

- **MILLER H.C.: "RADTECH 91" 1991 XP002255813 Seite 321 -Seite 324; Abbildung 3; Tabelle 1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue (Meth)acrylester von polyalkoxyliertem Glycerin, ein vereinfachtes Verfahren zur Herstellung dieser Ester und Verwendung der so erhältlichen Reaktionsgemische.

Quellbare Hydrogel-bildende Polymerisate, sogenannte Superabsorber (Super-Absorbing Polymers, SAP), sind aus dem Stand der Technik bekannt. Hierbei handelt es sich um Netzwerke flexibler hydrophiler Polymerisate, die sowohl ionischer als auch nichtionischer Natur sein können. Diese sind in der Lage, wässrige Flüssigkeiten unter Bildung eines Hydrogels zu absorbieren und zu binden und werden daher bevorzugt für die Herstellung von Tampons, Windeln, Damenbinden, Inkontinenzartikeln, Trainingsunterwäsche für Kinder, Schuheinlagen und anderen Hygieneartikeln bei der Absorption von Körperflüssigkeiten verwendet. Superabsorber weren außerdem in anderen Gebieten der Technik eingesetzt, bei denen Flüssigkeiten, insbesondere Wasser oder wässrige Lösungen absorbiert werden. Diese Gebiete sind z.B. Lagerung, Verpackung, Transport (Verpackungsmaterial wassersensitiver Artikel, wie z.B. Blumentransport, Schock Protection); Lebensmittelsektor (Transport von Fisch, Frischfleisch; Absorption von Wasser, Blut in Frischfisch/-Fleisch-Verpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textil (Handschuhe, Sportbekleidung, Feuchtigkeitsregulation in Textilien, Schuheinlagen); chem.-techn. Anwendungen (Katalysator für org. Reaktionen, Immobilisierung großer funktioneller Moleküle (Enzyme), Adhesiv für Agglomerationen, Wärmespeicher, Filtrationshilfsmittel, hydrophile Komponente in Polymerlaminaten, Dispergiermittel, Verflüssiger); Bau- und Konstruktionswesen, Installation (Pulverspritzguss, lehmbasierende Putze, Vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freitsetzung von Wirkstoffen an Pflanzen); im Feuerschutz (Funkenflug) (Abdecken von Häusern bzw. Bedecken von Hauswänden mit SAP Gel, da das Wasser eine sehr hohe Wärmekapazität hat, kann ein Entflammen verhindert werden; Versprühen von SAP Gel bei Bränden wie z.B. Waldbränden); Coextrusionsmittel in thermoplastischen Polymeren (Hydrophilierung von Mehrschichtfolien); Herstellung von Folien und thermoplastischen Formkörpern, die Wasser absorbieren können (z.B. Regen- und Tauwasser speichernde Folien für die Landwirtschaft); SAP haltige Folien zum Frischhalten von Obst und Gemüse, die in feuchte Folien verpackt werden können; der SAP speichert vom Obst und Gemüse abgegebenes Wasser ohne Bildung von Kondensationstropfen und gibt das Wasser teilweise an das Obst und Gemüse wieder ab, so dass weder Faulen noch Verwelken einritt; SAP-Polystyrol Coextrudate z.B. für Lebensmittelverpackungen wie Fleisch, Fisch, Geflügel, Obst und Gemüse); Trägersubstanz in Wirkstoffformulierungen (Pharma, Pflanzenschutz). In den Hygieneartikeln befinden sich die Superabsorber in aller Regel im sog. absorbent core, der als weitere Materialien unter anderem Fasern (Cellulosefasern) umfasst, die als eine Art Flüssigkeitsreservoir die spontan beaufschlagten Flüssigkeitsmengen zwischenspeichern und eine gute Kanalisation der Körperflüssigkeiten im absorbent core hin zum Superabsorber gewährleisten sollen.

Der aktuelle Trend im Windelaufbau besteht darin, dünnere Konstruktionen mit reduziertem Cellulosefaseranteil und erhöhtem Hydrogelanteil herzustellen. Mit dem Trend zu immer dünner werdenden Windelkonstruktionen hat sich das Anforderungsprofil an die wasserquellbaren hydrophilen Polymeren über die Jahre deutlich verändert. Während zu Beginn der Entwicklung hochsaugfähiger Hydrogele zunächst allein das sehr hohe Quellvermögen in Vordergrund stand, hat sich später gezeigt, dass auch die Fähigkeit des Superabsorbers zur Flüssigkeitsweiterleitung und -verteilung von entscheidender Bedeutung ist. Es hat sich gezeigt, dass herkömmliche Superabsorber an der Oberfläche bei Benetzung mit Flüssigkeit stark anquellen, so dass der Flüssigkeitstransport ins Partikelinnere stark erschwert oder ganz unterbunden wird. Diese Eigenart des Superabsorbers wird auch als "Gelblocking" bezeichnet. Aufgrund der höheren Beladung des Hygieneartikels (Polymer pro Flächeneinheit) darf das Polymer im gequollenen Zustand keine Sperrschicht für nachfolgende Flüssigkeit bilden. Weist das Produkt gute Transporteigenschaften auf, so kann eine optimale Ausnutzung des gesamten Hygieneartikels gewährleistet werden. Das Phänomen des Gelblockings wird somit unterbunden, das im Extremfall zum Austritt der Flüssigkeit, der sog. Leakage des Hygieneartikels führt. Die Flüssigkeitsweiterleitung und -verteilung ist also bei der anfänglichen Absorption von Körperflüssigkeiten von entscheidender Bedeutung.

Gute Transporteigenschaften besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch eine höhere Vernetzung erreicht, wodurch allerdings die Retention des Produktes verringert wird. Eine elegante Methode zur Erhöhung der Gelfestigkeit stellt die Oberflächennachvernetzung dar. Bei diesem Verfahren werden getrocknete Superabsorber mit durchschnittlicher Vernetzungsdichte einer zusätzlichen Vernetzung unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Superabsorberschale sinkt, weist der Kern der Superabsorberpartikel durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass der Effekt des Gelblockings auftritt. Es ist durchaus wünschenswert, dass die Gesamtkapazität des Superabsorbers nicht spontan, sondern zeitversetzt ausgeschöpft wird. Da der Hygieneartikel in der Regel mehrmals mit Urin beaufschlagt wird, muss das Absorptionsvermögen des Superabsorbers sinnvollerweise nicht nach der ersten Disposition erschöpft sein.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Hydrogele werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie z.B. Rewet in der Windel und AUL, werden hydrophile, hochquellfähige Hydrogele im allgemeinen oberflächen- oder gelnachvernetzt. Diese Nachvernetzung ist dem Fachmann an sich bekannt und erfolgt bevorzugt in wässriger Gelphase oder als Oberflächennachvernetzung der gemahlenen und abgesiebten Polymerpartikel.

EP 238050 offenbart als mögliche Innenvernetzer für Superabsorber zwei- bzw. dreifach mit Acrylsäure oder Methacrylsäure veresterte Anlagerungsprodukte von Ethylenoxid und/oder Propylenoxid an Trimethylolpropan.

US 5482640 offenbart Aminoacrylate, die durch Addition aliphatisch gebundener Aminogruppen von Mono- oder Diaminen mit einem Molgewicht von 31 bis 300 an olefinische Doppelbindungen von Polyol-Acrylestern, die zwar Ethylenoxideinheiten, jedoch keine Proylenoxideinheiten enthalten, erzeugt werden.

H.C. Miller berichtete auf der "Radtec Asia 91", 15-18.04. 1994 (Conference Proceedings, S. 321-324) über trifunktionelle Acrylate, darunter Polyol-Acrylester, die von propoxyliertem, jedoch nicht ethoxyliertem Glycerin abgeleitet sind.

WO 00/44734 A1 lehrt ebenfalls trifunktionelle Acrylate auf Basis propoxylierten Glycerins, deren Umsetzungsprodukte mit zyklischen sekundären Aminen als Bestandteile strahlungshärtbarer Lacke verwendet werden.

Kommerziell erhältlich ist z.B. von Sartomer (Exton, PA, USA) unter den angegebenen Handelsnamen Trimethylolpropantriacrylat (SR 351), dreimal einfach ethoxyliertes Trimethylolpropantriacrylat (SR 454), dreimal zweifach ethoxyliertes Trimethylolpropantriacrylat (SR 499), dreimal dreifach ethoxyliertes Trimethylolpropantriacrylat (SR 502), dreimal fünffach ethoxyliertes Trimethylolpropantriacrylat (SR 9035) und insgesamt 20-fach ethoxyliertes Trimethylolpropantriacrylat (SR 415). Propoxylierte Trimethylolpropane Triacrylate sind unter den Handelsnamen SR 492 (dreimal 1 PO pro TMP) und CD 501 (dreimal 2 PO pro TMP) erhältlich. Als Glycerin Derivate sind unter CD 9021 hoch propoxyliertes Glycerin Triacrylat und unter CD 9020 Tripropoxyglycerintriacrylat im Handel.

Aus WO 93/21237 sind (Meth)acrylate von alkoxylierten mehrwertigen $C_2$ - $C_{10}$-Kohlenwasserstoffen als Vernetzer bekannt. Verwendet wurden Trimethylpropanvernetzer, die SR 351, SR 454, SR 502, SR 9035 und SR 415 entsprechen. Diese Vernetzer weisen 0, 3, 9, 15 oder 20 EO Einheiten pro TMP auf. Vorteilhaft sind laut WO 93/21237 3-mal 2 bis 7 EO Einheiten pro TMP, insbesondere 3-mal 4 bis 6 EO Einheiten pro TMP.

Nachteilig an diesen Verbindungen ist, dass zur zumindest teilweisen Abtrennung von Einsatzstoffen und Nebenprodukten - die in der genannten Schrift eingesetzten Vernetzer weisen einen Gehalt an Acrylsäure von weniger als 0,1 Gew% auf - aufwendige Reinigungsoperationen erforderlich sind.

Ethoxylierte Trimethylolpropantri(meth)acrylate werden in der Patentliteratur immer wieder als Innenvernetzer erwähnt, wobei nur die kommerziell von Sartomer erhältlichen TMP Derivate verwendet werden, so z.B. in WO 98/47951 Trimethylolpropanetriethoxylat-triacrylat, in WO 01/41818 Sartomer #9035 als sogenanntes hoch ethoxyliertes Trimethylol Propan Triacrylat (HeTMPTA) und in WO 01/56625 SR 9035 und SR-492.

Die Herstellung solcher höheren (Meth)acrylsäureester durch säurekatalysierte Veresterung von (Meth)acrylsäure mit den entsprechenden Alkoholen in Gegenwart eines Inhibitors/Inhibitorsystems sowie gegebenenfalls eines Lösungsmittels, wie z.B. Benzol, Toluol, Cyclohexan, ist allgemein bekannt.

Da bekanntlich der Bildung des Esters aus (Meth)acrylsäure und Alkohol eine Gleichgewichtsreaktion zugrunde liegt, wird, um wirtschaftliche Umsätze zu erzielen, in der Regel ein Einsatzstoff im Überschuss eingesetzt und/oder das gebildete Veresterungswasser und/oder der Zielester aus dem Gleichgewicht entfernt.

Daher wird bei der Herstellung der höheren (Meth)acrylsäureester in der Regel das Reaktionswasser entfernt und meist ein Überschuss an (Meth)acrylsäure eingesetzt.

US 4187 383 beschreibt ein Veresterungsverfahren von (Meth)acrylsäure mit organischen Polyolen bei einer Reaktionstemperatur von 20 bis 80 °C mit einem Äquivalent-Überschuss von 2 bis 3 : 1.

Nachteilig an diesem Verfahren ist, dass durch die niedrige Reaktionstemperatur die Reaktionszeiten bis zu 35 Stunden betragen und der Überschuss Säure im Reaktionsgemisch durch eine Neutralisation mit anschließender Phasentrennung entfernt wird.

WO 2001/14438 (Derwent-Abstract Nr. 2001-191644/19) und WO 2001/10920 (Chemical Abstracts 134:

163502) beschreiben Verfahren zur Veresterung von (Meth)acrylsäure mit Polyalkylenglykolmonoalkylethern im Verhältnis 3:1 - 50:1 in Gegenwart von Säuren und Polymerisationsinhibitoren und, nach Desaktivierung des sauren Katalysators, Copolymerisation des Rückstandes aus (Meth)acrylsäureester und (Meth)acrylsäure bei pH 1,5 - 3,5, sowie dessen Verwendung als Zementadditiv.

Nachteilig an diesen Verfahren ist, dass es auf Polyalkylenglykolmonoalkylether beschränkt ist, dass der Katalysator desaktiviert werden muß und dass derartige Copolymerisate nicht als Vernetzer für Hydrogele eingesetzt werden können, da sie lediglich eine Funktionalität aufweisen.

Es bestand die Aufgabe, weitere Verbindungen zur Verfügung zu stellen, die als Radikalvernetzer für Polymere, insbesondere für Superabsorber verwendet werden können und das Herstellungsverfahren für Substanzen, die als Radikalvernetzer für Superabsorber verwendbar sind, zu vereinfachen.

Die Aufgabe wird gelöst durch das zur Verfügung stellen von einem Ester F der Formel Ia:

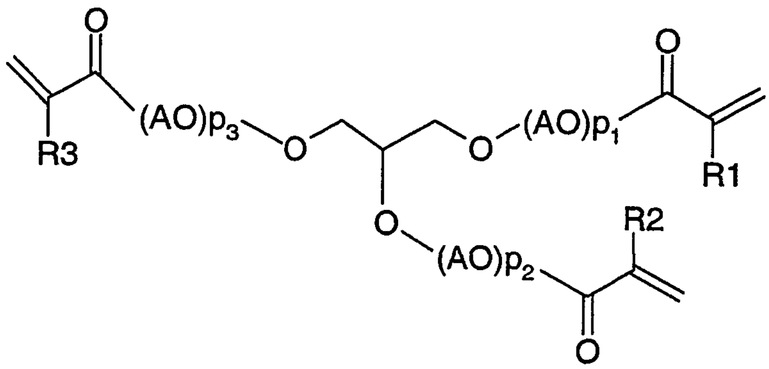

mit
AO bedeutet für jedes AO unabhängig voneinander EO oder PO,
wobei EO bedeutet O-CH2-CH2-,
PO bedeutet unabhängig voneinander O-CH2-CH(CH3)- oder O-CH(CH3)-CH2-
p1 + p2 + p3 ist 3, 4 oder 5,
R1, R2, R3 unabhängig voneinander H oder CH3.

Die EO bzw. PO Einheiten, sind so eingebaut, dass Polyether und keine Peroxide entstehen.

Bevorzugt sind die obigen Ester F, wobei AO EO bedeutet.

Bevorzugt sind außerdem obige Ester F, wobei mindestens ein AO PO und mindestens ein weiteres AO EO bedeutet.

Desweiteren wird die Aufgabe durch Ester F der Formel Ib gelöst:

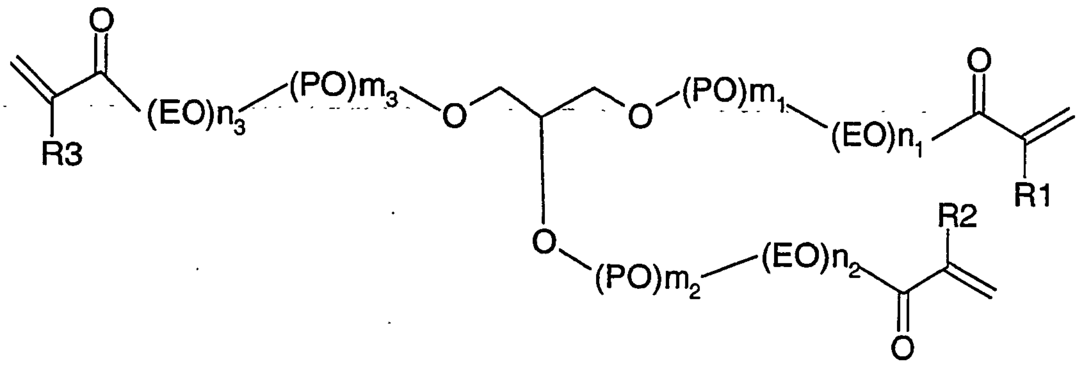

mit
EO bedeutet O-CH2-CH2-
PO bedeutet unabhängig voneinander O-CH2-CH(CH3)- oder O-CH(CH3)-CH2-
m1 + m2 + m3 + n1 + n2 + n3 ist 3, 4 oder 5,
m1 + m2 + m3 ist 1, 2, 3, oder 4,

R1, R2, R3 unabhängig voneinander H oder CH3.

Oder auch durch Ester F der Formel Ic:

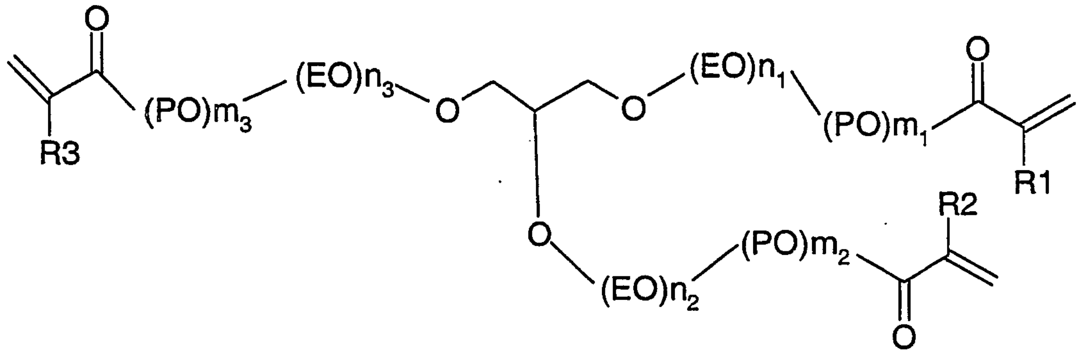

mit
EO bedeutet O-CH2-CH2-
PO bedeutet unabhängig voneinander O-CH2-CH(CH3)- oder O-CH(CH3)-CH2-
m1 +m2+m3+n1 + n2 + n3 ist 3, 4 oder 5,
m1 + m2 + m3 ist 1, 2, 3, oder 4,
R1, R2, R3 unabhängig voneinander H oder CH3.

Bei den obigen Estern bevorzugt sind Ester F, wobei m1 + m2 + m3 + n1 + n2 + n3 oder p1 + p2 + p3 gleich 3 ist.

Bei den obigen Estern bevorzugt sind Ester F, wobei m1 + m2 + m3 + n1 + n2 + n3 oder p1 + p2 + p3 gleich 5 ist.

Besonders bevorzugt sind Ester F, wobei insgesamt 3 PO vorliegen.

Ganz besonders bevorzugt sind Ester F, wobei in jeder der 3 Alkoxyketten des Glycerins mindestens 1 PO vorliegt.

Ganz besonders bevorzugt sind Ester F, bei den R1, R2 und R3 identisch sind, insbesondere wenn R1, R2 und R3 H bedeuten.

Erfindungsgemäß können die Ester F der oben genannten Formel mit den angegebenen Bedeutungen zur Herstellung von wässrige Flüssigkeiten absorbierende Hydrogel-formende Polymere verwendet werden, insbesondere als Innenvernetzer.

Die weitere Aufgabe wird gelöst durch ein Verfahren zur Herstellung eines Esters F von alkoxyliertem Glycerin mit (Meth)acrylsäure, umfassend die Schritte

a) Umsetzung von alkoxyliertem Glycerin mit (Meth)acrylsäure in Anwesenheit mindestens eines Veresterungskatalysators C und mindestens eines Polymerisationsinhibitors D sowie gegebenenfalls eines mit Wasser ein Azeotrop bildenden Lösungsmittels E unter Bildung eines Esters F,
b) gegebenenfalls Entfernen zumindest eines Teils des in a) entstehenden Wassers aus dem Reaktionsgemisch, wobei b) während und/oder nach a) erfolgen kann,
f) gegebenenfalls Neutralisation des Reaktionsgemischs,
h) falls ein Lösungsmittel E eingesetzt wurde gegebenenfalls Entfernen dieses Lösungsmittels durch Destillation und/oder
i) Strippen mit einem unter den Reaktionsbedingungen inerten Gas.

Bevorzugt beträgt hierbei

- der molare Überschuss (Meth)acrylsäure zu alkoxyliertem Glycerin 3,15:1 und
- verbleibt die in dem nach dem letzten Schritt erhaltenen Reaktionsgemisch enthaltene, gegebenenfalls neutralisierte (Meth)acrylsäure im wesentlichen im Reaktionsgemisch.

Unter (Meth)acrylsäure wird bei der vorliegenden Erfindung Methacrylsäure, Acrylsäure oder Gemische aus Methacrylsäure und Acrylsäure verstanden. Bevorzugt ist Acrylsäure.

Wird der Ester F in Reinform gewünscht kann er durch bekannte Auftrennungsverfahren gereinigt werden.

Der molare Überschuss von (Meth)acrylsäure zu alkoxyliertem Glycerin beträgt mindestens 3,15:1, bevorzugt mindestens 3,3:1, besonders bevorzugt mindestens 3,75:1, ganz besonders bevorzugt mindestens 4,5:1 und insbesondere mindestens 7,5:1.

In einer bevorzugten Ausführungsform wird (Meth)acrylsäure in einem Überschuss von beispielsweise größer als 15:1, bevorzugt größer als 30:1, besonders bevorzugt größer als 60:1, ganz besonders bevorzugt größer 150:1, insbesondere größer 225:1 und speziell größer als 300:1 eingesetzt.

Die so erhältlichen Veresterungsprodukte können im wesentlichen ohne weitere Reinigung, besonders ohne wesentliche Abtrennung des Überschusses an (Meth)acrylsäure und des Gehalts an Veresterungskatalysator C, als Radikalvernetzer in Hydrogelen eingesetzt werden.

Unter Vernetzung wird in dieser Schrift wenn nicht anders erwähnt die Radikalvernetzung (Gelvernetzung, Innenvernetzung, Quervernetzung von linearem oder schwach vernetzten Polymer) als verstanden. Diese Vernetzung kann über radikalische oder kationische Polymerisationsmechanismen oder andere, beispielsweise Michael-Addition, Ver- oder Umesterungsmechanismen erfolgen, bevorzugt durch radikalische Polymerisation.

Wässrige Flüssigkeiten absorbierende Hydrogel-formende Polymere sind bevorzugt solche mit einer Absorption von destilliertem Wasser von mindestens dem Eigengewicht, bevorzugt dem 10-fachem Eigengewicht, insbesondere dem 20-fachem Eigengewicht, diese Absorption wird bevorzugt auch unter einem Druck von 0,7 psi erreicht.

Erfindungsgemäß einsetzbare alkoxyliertem Glycerin haben die Struktur wie in Formel IIa, IIb oder IIc

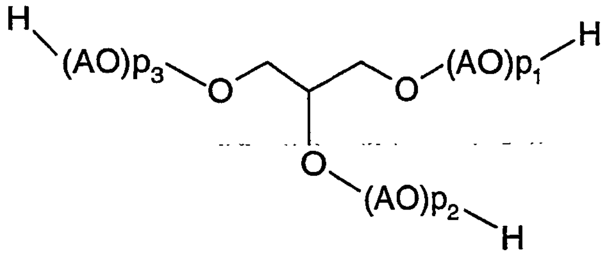

IIa,

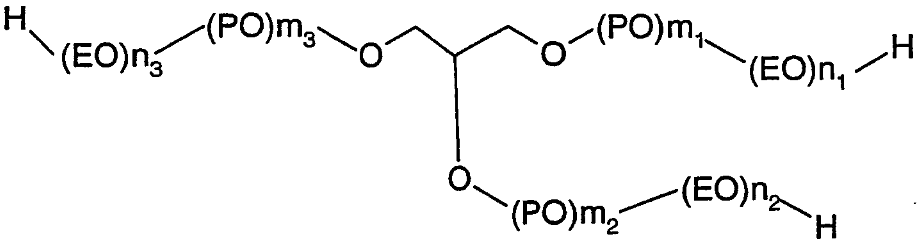

IIb,

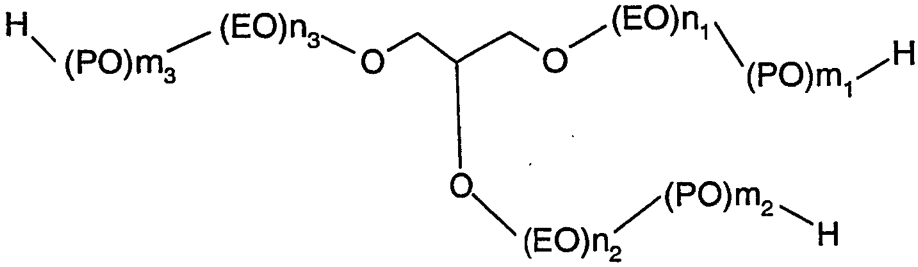

IIc,

wobei AO, EO, PO, n1, n2, n3, m1, m2, m3, p1, p2, p3 die bei den Estern angegebenen Bedeutung besitzen.

Die Umsetzung von Glycerin mit einem Alkylenoxid ist dem Fachmann an sich bekannt. Mögliche Durchführungsformen finden sich in Houben-Weyl, Methoden der Organischen Chemie, 4. Auflage, 1979, Thieme Verlag Stuttgart, Hrsg. Heinz Kropf, Band 6/1 a, Teil 1, Seiten 373 bis 385.

Zur Herstellung von Verbindungen der Formel II wird z.B. zuerst das Glycerin mit EO abreagiert und dann anschließend mit PO umgesetzt oder erst PO und dann EO oder PO und EO werden gleichzeitig eingesetzt.

Dies kann z.B. geschehen in dem etwa 77 g Glycerin mit 0,5 g KOH, 45 % in Wasser, in einem Autoklaven vorgelegt wird und zusammen bei 80°C und reduziertem Druck (ca. 20 mbar) entwässert wird. Dann wird bei 120 bis 130°C die entsprechende Menge an Propylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei 120 °C nachgerührt. Anschließend wird die entsprechende Menge an Ethylenoxid bei 145 bis 155°C über längere Zeit bei erhöhtem Druck zudosiert und ebenfalls abreagieren gelassen. Nach Spülen mit Inertgas und Abkühlen auf 60 °C wird der Katalysator durch Zugabe von Natriumpyrophosphat und anschließende Filtration abgetrennt.

An die Viskosität der erfindungsgemäß einsetzbaren Polyalkohole werden keine besonderen Ansprüche gestellt, außer dass sie bei einer Temperatur bis zu ca. 80 °C problemlos pumpbar sein sollten, bevorzugt sollten sie eine Viskosität unter 1000 mPas aufweisen, bevorzugt unter 800 mPas und ganz besonders bevorzugt unter 500 mPas.

Erfindungsgemäß einsetzbare Veresterungskatalysatoren C sind Schwefelsäure, Aryl- oder Alkylsulfonsäuren oder Gemische davon. Beispiele für Arylsulfonsäuren sind Benzolsulfonsäure, para-Toluolsulfonsäure oder Dodecylbenzolsulfonsäure, Beispiele für Alkylsulfonsäuren sind Methansulfonsäure, Ethansulfonsäure oder Trifluormethansulfonsäure. Auch stark saure Ionentauscher oder Zeolithe sind als Veresterungskatalysatoren einsetzbar. Bevorzugt sind Schwefelsäure und Ionentauscher.

Erfindungsgemäß einsetzbare Polymerisationsinhibitoren D sind beispielsweise Phenole wie Alkylphenole, beispielsweise o-, m- oder p-Kresol (Methylphenol), 2-tert.-Butyl-4-methylphenol, 6-tert.-Butyl-2,4-dimethyl-phenol, 2,6-Di-tert.-Butyl-4-methylphenol, 2-tert.-Butylphenol, 4-tert.-Butylphenol, 2,4-di-tert.-Butylphenol, 2-Methyl-4-tert.-Butylphenol, 4-tert.-Butyl-2,6-dimethyfphenol, oder 2,2'-Methylen-bis-(6-tert.-butyl-4-methylphenol), 4,4'-Oxydiphenyl, 3,4-Methylendioxydiphenol (Sesamol), 3,4-Dimethylphenol, Hydrochinon, Brenzcatechin (1,2-Dihydroxybenzol), 2-(1'-Methylcyclohex-1'-yl)-4,6-dimethylphenol, 2- oder 4-(1'-Phenyl-eth-1'-yl)-phenol, 2-tert-Butyl-6-methylphenol, 2,4,6-Tris-tert-Butylphenol, 2,6-Di-tert.-butylphenol, 2,4-Di-tert.-butylphenol, 4-tert.-Butylphenol, Nonylphenol [11066-49-2], Octylphenol [140-66-9], 2,6-Dimethylphenol, Bisphenol A, Bisphenol F, Bisphenol B, Bisphenol C, Bisphenol S, 3,3',5,5'-Tetrabromobisphenol A, 2,6-Di-tert-Butyl-p-kresol, Koresin® der BASF AG, 3,5-Di-tert-Butyl-4-hydroxybenzoesäuremethylester, 4-tert-Butylbrenzcatechin, 2-Hydroxybenzylalkohol, 2-Methoxy-4-methylphenol, 2,3,6-Trimethylphenol, 2,4,5-Trimethylphenol, 2,4,6-Trimethylphenol, 2-Isopropylphenol, 4-Isopropylphenol, 6-Isopropyl-m-Kresol, n-Octadecyl-beta-(3,5-di-tert-butyl-4-hydroxyphenyl)propionat, 1,1,3-Tris-(2-methyl-4-hydroxy-5-tert-butylphenyl)butan, 1,3,5-Trimethyl-2,4,6-tris-(3,5-di-tert-butyl-4-hydroxybenzyl)benzol, 1,3,5,-Tris-(3,5-di-tert-butyl-4-hydroxybenzyl)isocyanurat, 1,3,5,-Tris-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat oder Pentaerythrit-tetrakis-[beta-(3,5,-di-tert-butyl-4-hydroxyphenyl)-propionat], 2,6-Di-*tert*.-butyl-4-dimethylaminomethyl-phenol, 6-*sek*.-Butyl-2,4-dinitrophenol, Irganox® 565, 1141, 1192, 1222 und 1425 der Firma Ciba Spezialitätenchemie, 3-(3',5'-Di-*tert*-butyl-4'-hydroxyphenyl)propionsäureoctadecylester, 3-(3',5'-Di-*tert*.-butyl-4'-hydroxyphenyl)propionsäurehexadecylester, 3-(3',5'-Di-*tert*.-butyl-4'-hydroxyphenyl)propionsäureoctylester, 3-Thia-1,5-pentandiol-bis-[(3',5'-di-*tert*.-butyl-4'-hydroxyphenyl)propionat], 4,8-Dioxa-1,11-undecandiol-bis-[(3',5'-di-*tert*.-butyl-4'-hydroxyphenyl)propionat], 4,8-Dioxa-1,11-undecandiol-bis-[(3'-*tert*.-butyl-4'-hydroxy-5'-methylphenyl)propionat], 1,9-Nonandiol-bis-[(3',5'-di-*tert*.-butyl-4'-hydroxyphenyl)propionat], 1,7-Heptandiamin-bis[3-(3',5'-di-*tert*.-butyl-4'-hydroxyphenyl)propionsäureamid], 1,1-Methandiamin-bis[3-(3',5'-di-*tert*.-butyl-4'-hydroxyphenyl)propionsäureamid], 3-(3',5'-di-*tert*-Butyl-4'-hydroxyphenyl)propionsäurehydrazid, 3-(3',5'-di-Methyl-4'-hydroxyphenyl)propionsäurehydrazid, Bis(3-*tert*.-Butyl-5-ethyl-2-hydroxy-phen-1-yl)methan, Bis(3,5-di-*tert*.-Butyl-4-hydroxy-phen-1-yl)methan, Bis[3-(1'-methylcyclohex-1'-yl)-5-methyl-2-hydroxy-phen-1-yl]methan, Bis(3-*tert*.-Butyl-2-hydroxy-5-methyl-phen1-yl)methan, 1,1-Bis(5-*tert*.-Butyl-4-hydroxy-2-methyl-phen-1-yl)ethan, Bis(5-*tert*.-Butyl-4-hydroxy-2-methyl-phen-1-yl)sulfid, Bis (3-*tert*.-Butyl-2-hydroxy-5-methyl-phen-1-yl)sulfid, 1,1-Bis(3,4-Dimethyl-2-hydroxy-phen-1-yl)-2-methylpropan, 1,1-Bis (5-*tert*.-Butyl-3-methyl-2-hydroxy-phen-1-yl)-butan, 1,3,5-Tris[1'-(3",5"-di-*tert*-Butyl-4"-hydroxyphen-1"-yl)-meth-1'-yl]-2,4,6-trimethylbenzol, 1,1,4-Tris(5'-*tert*-Butyl-4'-hydroxy-2'-methyl-phen-1'-yl)butan, Aminophenole, wie z.B. para-Aminophenol, Nitrosophenole, wie z.B. para-Nitrosophenol, p-Nitroso-o-Kresol, Alkoxyphenole, beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol, 3,5-Di-tert-butyl-4-hydroxyanisol, 3-Hydroxy-4-methoxybenzylalkohol, 2,5-Dimethoxy-4-hydroxybenzylalkohol (Syringaalkohol), 4-Hydroxy-3-methoxybenzaldehyd (Vanillin), 4-Hydroxy-3-ethoxybenzaldehyd (Ethylvanillin), 3-Hydroxy-4-methoxybenzaldehyd (Isovanillin), 1-(4-Hydroxy-3-methoxy-phenyl)ethanon (Acetovanillon), Eugenol, Dihydroeugenol, Isoeugenol, phenyl)ethanon (Acetovanillon), Eugenol, Dihydroeugenol, Isoeugenol, Tocopherole, wie z.B. alpha-, beta-, gamma-, delta- und epsilon-Tocopherol, Tocol, alpha-Tocopherolhydrochinon, sowie 2,3-Dihydro-2,2-dimethyl-7-hydroxybenzofuran (2,2-Dimethyl-7-hydroxycumaran), Chinone und Hydrochinone wie Hydrochinon oder Hydrochinonmonomethylether, 2,5-Di-*tert*.-Butylhydrochinon, 2-Methyl-p-hydrochinon, 2,3-Dimethylhydrochinon, Trimethylhydrochinon, 4-Methylbrenzcatechin, tert-Butylhydrochinon, 3-Methylbrenzcatechin, Benzochinon, 2-Methyl-p-hydrochinon, 2,3-Dimethylhydrochinon, Trimethylhydrochinon, 3-Methylbrenzcatechin, 4-Methylbrenzcatechin, tert-Butylhydrochinon, 4-Ethoxyphenol, 4-Butoxyphenol, Hydrochinonmonobenzylether, p-Phenoxyphenol, 2-Methylhydrochinon, 2,5-Di-tert.-Butylhydrochinon, Tetramethyl-p-benzochinon, Diethyl-1,4-cyclohexandion-2,5-dicarboxylat, Phenyl-p-benzochinon, 2,5-Dimethyl-3-benzyl-p-benzochinon, 2-Isopropyl-5-methyl-p-benzochinon (Thymochinon), 2,6-Diisopropyl-p-benzochinon, 2,5-Dimethyl-3-hydroxy-p-benzochinon, 2,5-Dihydroxy-p-benzochinon, Embelin, Tetrahydroxy-p-benzochinon, 2,5-Dimethoxy-1,4-benzochinon, 2-Amino-5-methyl-p-benzochinon, 2,5-Bisphenylamino-1,4-benzochinon, 5,8-Dihydroxy-1,4-naphthochinon, 2-Anilino-1,4,naphthochinon, Anthrachinon, N,N-Dimethylindoanilin, N,N-Diphenyl-p-benzochinondiimin, 1,4-Benzochinondioxim, Coerulignon, 3,3'-Di-tert-butyl-5,5'-dimethyldiphenochinon, p-Rosolsäure (Aurin), 2,6-Di-tert-butyl-4-benzyliden-benzochinon, 2,5-Di-tert.-Amylhydrochinon, N-Oxyle wie 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-tetramethyl-piperidin-N-oxyl, 4,4',4"-Tris(2,2,6,6-tetramethyl-piperidin-N-oxyl)-phosphit, 3-Oxo-2,2,5,5-tetramethylpyrrolidin-N-oxyl, 1-Oxyl-2,2,6,6-tetramethyl-4-methoxypiperidin, 1-Oxyl-2,2,6,6-tetramethyl-4-trimethylsilyloxypiperidin, 1-Oxyl-2,2,6,6-tetramethylpiperidin-4-yl-2-ethylhexanoat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-(4-tert-butyl)benzoat, Bis(1-Oxyl-2,2,6,6-tetramethylpiperidin-4-yl)-succinat, Bis(1-Oxyl-2,2,6,6-tetramethylpiperidin-4-yl)-adipat, 1,10-Dekandisäure-bis(1-Oxyl-2,2,6,6-tetramethylpiperidin-4-yl)ester, Bis(1-Oxyl-2,2,6,6-tetramethylpiperidin-4-yl)-n-butylmalonat, Bis(1-Oxyl-2,2,6,6-tetramethylpiperidin-4-yl)-phthalat, Bis (1-Oxyl-2,2,6,6-tetramethylpiperidin-4-yl)-isophthalat, Bis(1-Oxyl-2,2,6,6-tetramethylpiperidin-4-yl)-terephthalat, Bis(1-Oxyl-2,2,6,6-tetramethylpiperidin-4-yl)-hexahydroterephthalat, N,N'-Bis(1-Oxyl-2,2,6,6-tetramethylpiperidin-4-yl)-adipinamid, N-(1-Oxyl-2,2,6,6-tetramethylpiperidin-4-yl)-caprolactam, N-(1-Oxyl-2,2,6,6-tetramethylpiperidin-4-yl)-dodecylsuccinimid, 2,4,6-Tris-[N-butyl-N-(1-Oxyl-2,2,6,6-tetramethylpiperidin-4-yl]triazin, N,N'-Bis(1-Oxyl-2,2,6,6-tetramethylpiperidin-4-yl)-N,N'-bis-formyl-1,6-diaminohexan, 4,4'-Ethylenbis(1-Oxyl-2,2,6,6-tetramethylpiperazin-3-on) aromatische Amine wie Phenylendiamine, N,N-Diphenylamin, N-Nitroso-diphenylamin, Nitrosodiethylanilin, N,N'-Dialkyl-paraphenylendiamin, wobei die Alkylreste gleich oder verschieden sein können und jeweils unabhängig voneinander aus 1 bis 4 Kohlenstoffatome bestehen und geradkettig oder verzweigt sein können, beispielsweise N,N'-Di-iso-butyl-p-phenylendiamin, N,N'-Di-iso-propyl-p-phenylendiamin, Irganox 5057 der Firma Ciba Spezialitätenchemie, N,N'-Di-iso-butyl-p-phenylendiamin, N,N'-Di-isopropyl-p-phenylendiamin, p-Phenylendiamin, N-Phenyl-p-Phenylendiamin, N,N'-Diphenyl-p-phenylendiamin, N-Isopropyl-N-phenyl-p-phenylendiamin, N,N'Di-sec-butyl-p-phenylendiamin (Kerobit® BPD der BASF AG), N-Phenyl-N'-isopropyl-p-phenylendiamin (Vulkanox® 4010 der Bayer AG), N-(1,3-Dimethylbutyl)-N'-phenyl-p-phenylendiamin, N-Phenyl-2-naphthylamin, Imoinodibenzyl, N,N'-Diphenylbenzidin, N-Phenyltetraanilin, Acridon, 3-Hydroxydiphenylamin, 4-Hydroxydiphenylamin, Hydroxylamine wie N,N-Diethylhydroxylamin, Harnstoffderivate wie Harnstoff oder Thioharnstoff, phosphorhaltige Verbindungen, wie Triphenylphosphin, Triphenylphosphit, Hypophosphorige Säure oder Triethylphosphit, schwefelhaltige Verbindungen, wie Diphenylsulfid, Phenothiazin oder Metallsalze, wiebeispielsweise Kupfer-, Mangan-, Cer-, Nickel-, Chrom-, -chlorid, -dithiocarbamat, -sulfat, -salicylat oder -acetat oder Gemische davon. Bevorzugt sind die genannten Phenole und Chinone, besonders bevorzugt sind Hydrochinon, Hydrochinonmonomethylether, 2-tert.-Butyl-4-methylphenol, 6-tert.-Butyl-2,4-dimethyl-phenol, 2,6-Di-tert.-Butyl-4-methylphenol, 2,4-di-tert.-Butylphenol, Triphenylphosphit, Hypophosphorige Säure, $CuCl_2$ und Guajacol, ganz besonders bevorzugt sind Hydrochinon und Hydrochinonmonomethylether.

Besonders bevorzugt sind Hydrochinonmonomethylether, Hydrochinon, und Alkylphenole, gegebenenfalls in Kombination mit Tripehnylphosphit und/oder Hypophosphoriger Säure.

Zur weiteren Unterstützung der Stabilisierung kann ein sauerstoffhaltiges Gas, bevorzugt Luft oder ein Gemisch aus Luft und Stickstoff (Magerluft) anwesend sein.

Unter den aufgeführten Stabilisatoren sind solche bevorzugt, die aerob sind, d.h. solche, die zur Entfaltung ihrer vollen Inhibitorwirkung die Anwesenheit von Sauerstoff erfordern.

Erfindungsgemäß einsetzbare Lösungsmittel E sind besonders solche, die sich zur azeotropen Entfernung des Reaktionswassers, falls gewünscht, eignen, vor allem aliphatische, cycloaliphatische und aromatische Kohlenwasserstoffe oder Gemische davon.

Vorzugsweise kommen n-Pentan, n-Hexan, n-Heptan, Cyclohexan, Methylcyclohexan, Benzol, Toluol oder Xylol zur Anwendung. Besonders bevorzugt sind Cyclohexan, Methylcyclohexan und Toluol.

Für die Veresterung können die dem Fachmann bekannten Herstell- und/oder Aufarbeitungsverfahren von mehrwertigen Alkoholen angewendet werden, beispie!sweise die eingangs erwähnten oder die in DE-A 199 41 136, DE-A 38 43 843, DE-A 38 43 854, DE-A 199 37 911, DE-A 199 29 258, EP-A 331 845, EP 554 651 oder US 4 187 383 beschriebenen.

Im allgemeinen kann die Veresterung wie folgt durchgeführt werden:

Die Veresterungsapparatur besteht aus einem gerührten Reaktor, bevorzugt aus einem Reaktor mit Umlaufverdampfer und einer aufgesetzten Destillationseinheit mit Kondensator und Phasentrenngefäß.

Bei dem Reaktor kann es sich beispielsweise um einen Reaktor mit Doppelwandheizung oder/und innenliegenden Heizschlangen handeln. Vorzugsweise wird ein Reaktor mit außenliegendem Wärmetauscher und Natur- oder Zwangsumlauf, d.h. unter Verwendung einer Pumpe, besonders bevorzugt Naturumlauf, bei dem der Kreislaufstrom ohne mechanische Hilfsmittel bewerkstelligt wird, eingesetzt.

Selbstverständlich kann die Reaktion auch in mehreren Reaktionszonen, beispielsweise einer Reaktorkaskade aus zwei bis vier, bevorzugt zwei bis drei Reaktoren durchgeführt werden.

Geeignete Umlaufverdampfer sind dem Fachmann bekannt und beispielsweise beschrieben in R. Billet, Verdampfertechnik, HTB-Verlag, Bibliographisches Institut Mannheim, 1965, 53. Beispiele für Umlaufverdampfer sind Rohrbündelwärmetauscher, Plattenwärmetauscher, etc.

Selbstverständlich können im Umlauf auch mehrere Wärmetauscher vorhanden sein.

Die Destillationseinheit ist von an sich bekannter Bauart. Dabei kann es sich um eine einfache Destillation handeln, die gegebenenfalls mit einem Spritzschutz ausgestattet ist, oder um eine Rektifikationskolonne. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern oder Geflechten bevorzugt.

In der Regel sind 5 bis 20 theoretische Böden ausreichend.

Der Kondensator und das Trenngefäß sind von herkömmlicher Bauart.

(Meth)acrylsäure und alkoxyliertes Glycerin werden in der Veresterung a) in der Regel im molaren Überschuss wie oben angegeben eingesetzt. Der eingesetzte Überschuss kann bis zu ca. 3000:1 betragen, falls gewünscht.

Als Veresterungskatalysatoren C kommen die oben angeführten in Frage.

Sie werden in der Regel in einer Menge von 0,1 - 5 Gew%, bezogen auf das Veresterungsgemisch, eingesetzt, bevorzugt 0,5 - 5, besonders bevorzugt 1 - 4 und ganz besonders bevorzugt 2 - 4 Gew%.

Falls erforderlich kann der Veresterungskatalysator aus dem Reaktionsgemisch mit Hilfe eines Ionenaustauschers entfernt werden. Der Ionenaustauscher kann dabei direkt in das Reaktionsgemisch gegeben und anschließend abfiltriert oder das Reaktionsgemisch kann über eine Ionenaustauscherschüttung geleitet werden.

Bevorzugt wird der Veresterungskatalysator im Reaktionsgemisch belassen. Handelt es sich jedoch bei dem Katalysator um einen Ionentauscher, so wird dieser bevorzugt entfernt, beispielsweise durch Filtration.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich einer Kolonne und/oder in einen Umlaufverdampfer eindosiert und/oder durch das Reaktionsgemisch und/oder über dieses geleitet.

Das Polymerisationsinhibitor(gemisch) D (wie oben angeführt) wird in einer Gesamtmenge von 0,01 - 1 Gew%, bezogen auf das Veresterungsgemisch, eingesetzt, bevorzugt 0,02 - 0,8, besonders bevorzugt 0,05 - 0,5 Gew%.

Das Polymerisationsinhibitor(gemisch) D kann beispielsweise als wässrige Lösung oder als Lösung in einem Edukt oder Produkt eingesetzt werden.

b) Das bei der Reaktion entstehende Reaktionswasser kann während oder nach der Veresterung a) abdestilliert werden, wobei dieser Vorgang durch ein mit Wasser ein Azeotrop bildendes Lösungsmittel unterstützt werden kann.

Als Lösungsmittel E zur azeotropen Entfernung des Reaktionswassers, falls gewünscht, eignen sich die oben angeführten Verbindungen.

Bevorzugt ist die Durchführung der Veresterung in Gegenwart eines Lösungsmittels.

Die eingesetzte Menge an Lösungsmittel beträgt 10 - 200 Gew%, vorzugsweise 20 - 100 Gew%, besonders bevorzugt 30 bis 100 Gew% bezogen auf die Summe von alkoxyliertem Glycerin und (Meth)acrylsäure.

Denkbar ist jedoch auch eine Durchführung ohne Schleppmittel, wie sie z.B. in der DE-A1 38 43 854, Sp. 2, Z. 18 bis Sp. 4, Z. 45 beschrieben ist, jedoch im Unterschied dazu mit den oben genannten Stabilisatoren.

Falls das im Reaktionsgemisch enthaltene Wasser nicht über ein azeotropbildendes Lösungsmittel entfernt wird, so ist es möglich, dieses über Strippen mit einem inerten Gas, bevorzugt einem sauerstoffhaltigen Gas, besonders bevorzugt mit Luft oder Magerluft zu entfernen, beispielsweise wie in der DE-A 38 43 843 beschrieben.

Die Reaktionstemperatur der Veresterung a) liegt allgemein bei 40 - 160 °C, bevorzugt 60 - 140°C und besonders bevorzugt 80 - 120°C. Die Temperatur kann im Reaktionsverlauf gleichbleiben oder ansteigen, bevorzugt wird sie im Reaktionsverlauf angehoben. In diesem Fall ist die Endtemperatur der Veresterung um 5 - 30 °C höher als die Anfangstemperatur. Die Temperatur der Veresterung kann durch Variation der Lösungsmittelkonzentration im Reaktionsgemisch bestimmt und geregelt werden, wie in DE-A 199 41 136 und der deutschen Anmeldung mit dem Aktenzeichen 100 63 175.4 beschrieben.

Falls ein Lösungsmittel eingesetzt wird, so kann dieses über die dem Reaktor aufgesetzte Destillationseinheit vom Reaktionsgemisch abdestilliert werden.

Das Destillat kann wahlweise entfernt werden, oder, nach Kondensation, in einen Phasentrennapparat geführt werden. Die so erhaltene wässrige Phase wird in der Regel ausgeschleust, die organische Phase kann als Rücklauf in die Destillationseinheit geführt und/oder direkt in die Reaktionszone geleitet werden und/oder in einen Umlaufverdampfer geführt werden, wie in der deutschen Patentanmeldung mit dem Aktenzeichen 100 63 175.4 beschrieben.

Bei Verwendung als Rücklauf kann die organische Phase, wie in der DE-A 199 41 136 beschrieben, zur Steuerung der Temperatur in der Veresterung verwendet werden.

Die Veresterung a) kann drucklos aber auch bei Überdruck oder Unterdruck durchgeführt werden, vorzugsweise wird bei normalem Druck gearbeitet.

Die Reaktionszeit beträgt in der Regel 2 - 20 Stunden, vorzugsweise 4 -15 und besonders bevorzugt 7 bis 12 Stunden.

Die Reihenfolge der Zugabe der einzelnen Reaktionskomponenten ist erfindungsgemäß nicht wesentlich. Es können alle Komponenten gemischt vorgelegt und anschließend aufgeheizt werden oder es können eine oder mehrere Komponenten nicht oder nur teilweise vorgelegt und erst nach dem Aufheizen zugegeben werden.

Die einsetzbare (Meth)acrylsäure ist in ihrer Zusammensetzung nicht beschränkt und kann beispielsweise folgende Komponenten aufweisen:

| | |
|---|---|
| (Meth)acrylsäure | 90 - 99,9 Gew% |
| Essigsäure | 0,05 - 3 Gew% |
| Propionsäure | 0,01 - 1 Gew% |
| Diacrylsäure | 0,01 - 5 Gew% |
| Wasser | 0,05 - 5 Gew% |
| Carbonylhaltige | 0,01 - 0,3 Gew% |
| Inhibitoren | 0,01 - 0,1 Gew% |
| Maleinsäure(-anhydrid) | 0,001 - 0,5 Gew% |

Die eingesetzte Roh-(Meth)acrylsäure ist in der Regel stabilisiert mit 200 - 600 ppm Phenothiazin oder anderen Stabilisatoren in Mengen, die eine vergleichbare Stabilisierung ermöglichen. Unter dem Ausfruck Carbonylhaltige werden hier beispielsweise Aceton und niedere Aldehyde, wie z.B. Formaldehyd, Acetaldehyd, Crotonaldehyd, Acrolein, 2- und 3- Furfural und Benazaldehyd, verstanden.

Unter Roh-(Meth)acrylsäure wird hier das (meth)acrylsäurehaltige Gemisch verstanden, das nach Absorption der Reaktionsgase der Propan/Propen/Acrolein- beziehungsweise Isobutan/Isobuten/Methacrolein-Oxidation in einem Absorptionsmittel und anschließender Abtrennung des Absorptionsmittels anfällt beziehungsweise das durch fraktionierende Kondensation der Reaktionsgase gewonnen wird.

Selbstverständlich kann auch Rein-(Meth)acrylsäure eingesetzt werden mit beispielsweise folgender Reinheit:

| | |
|---|---|
| (Meth)acrylsäure | 99,7 - 99,99 Gew% |
| Essigsäure | 50 - 1000 Gew.ppm |
| Propionsäure | 10 - 500 Gew.ppm |
| Diacrylsäure | 10 - 500 Gew.ppm |
| Wasser | 50 - 1000 Gew.ppm |
| Carbonylhaltige | 1 - 500 Gew.ppm |
| Inhibitoren | 1 - 300 Gew.ppm |
| Maleinsäure(-anhydrid) | 1 - 200 Gew.ppm |

Die eingesetzte Rein-(Meth)acrylsäure ist in der Regel stabilisiert mit 100 - 300 ppm Hydrochinonmonomethylether oder anderen Lagerstabilisatoren in Mengen, die eine vergleichbare Stabilisierung ermöglichen.

Unter reiner bzw. vorgereinigter (Meth)acrylsäure wird allgemein (Meth)acrylsäure verstanden, deren Reinheit mind. 99,5 Gew% beträgt und die im wesentlichen frei von den aldehydischen, anderen carbonylhaltigen und hochsiedenden Komponenten ist.

Die während der Veresterung abdestillierte wässrige Phase des über die aufgesetzte Kolonne, wenn vorhanden, abgetrennten Kondensats, die in der Regel 0,1-10 Gew% (Meth)acrylsäure enthalten kann, wird abgetrennt und ausgeschleust. Vorteilhaft kann die darin enthaltene (Meth)acrylsäure mit einem Extraktionsmittel, bevorzugt dem gegebenenfalls in der Veresterung verwendeten Lösungsmittel, beispielsweise mit Cyclohexan, bei einer Temperatur zwischen 10 und 40 °C und einem Verhältnis von wässriger Phase zu Extraktionsmittel von 1 : 5 - 30, bevorzugt 1 : 10 - 20, extrahiert und in die Veresterung zurückgeführt werden.

Zur weiteren Unterstützung des Umlaufs kann ein inertes Gas, bevorzugt ein sauerstoffhaltiges Gas, besonders bevorzugt Luft oder ein Gemisch aus Luft und Stickstoff (Magerluft) in den Umlauf, durch oder über das Reaktionsgemisch geleitet werden, beispielsweise in Mengen von 0,1 -1, bevorzugt 0,2 - 0,8 und besonders bevorzugt 0,3 - 0,7 $m^3/m^3h$, bezogen auf das Volumen des Reaktionsgemisches.

Der Verlauf der Veresterung a) kann durch Verfolgung der ausgetragenen Wassermenge und/oder die Abnahme der Carbonsäurekonzentration im Reaktor verfolgt werden.

Die Reaktion kann beispielsweise beendet werden, sobald 90 % der theoretisch zu erwartenden Wassermenge durch das Lösungsmittel ausgetragen worden sind, bevorzugt bei mindestens 95% und besonders bevorzugt bei mindestens 98%.

Das Ende der Reaktion kann beispielsweise dadurch festgestellt werden, dass im wesentlichen kein weiteres Reaktionswasser mehr über das Schleppmittel enfernt wird. Wird (Methacryl)säure zusammen mit dem Reaktionswasser ausgetragen, so ist deren Anteil beispielsweise durch Rücktitration eines Aliquots der wässrigen Phase bestimmbar.

Auf ein Entfernen des Reaktionswasers kann beispielsweise verzichtet werden, wenn die (Methacryl)säure in einem hohen stöchiometrischen Überschuss eingesetzt wird, beispielsweise von mindestens 4,5:1, bevorzugt mindestens 7,5:1 und ganz besonders bevorzugt mindestens 15:1. In diesem Fall verbleibt ein wesentlicher Teil der entstandenen Wassermenge im Reaktionsgemisch. Während oder nach der Reaktion wird lediglich der Anteil an Wasser aus dem Reaktionsgemisch entfernt, der durch die Flüchtigkeit bei der angelegten Temperatur bestimmt ist und es werden darüberhinaus keine Maßnahmen zur Abtrennung des entstandenen Reaktionswassers durchgeführt. So können beispielsweise mindestens 10 Gew% des entstandenen Reaktionswassers im Reaktionsgemisch verbleiben, bevorzugt mindestens 20 Gew%, besonders bevorzugt mindestens 30 Gew%, ganz besonders bevorzugt mindestens 40 und insbesondere mindestens 50 Gew%.

c) Nach Beendigung der Veresterung kann das Reaktorgemisch auf übliche Weise auf eine Temperatur von 10 bis 30 °C abgekühlt werden und gegebenenfalls durch Zugabe von Lösungsmittel, das das gleiche wie das gegebenenfalls zur azeotropen Entfernung von Wasser verwendete Lösungsmittel oder ein anderes sein kann, eine beliebige Zielesterkonzentration eingestellt werden.

In einer weiteren Ausführungsform kann die Reaktion mit einem geeigneten Verdünnungsmittel G gestoppt werden und auf eine Konzentration von beispielsweise 10 - 90 Gew%, bevorzugt 20 - 80 %, besonders bevorzugt 20 bis 60%, ganz besonders bevorzugt 30 bis 50% und insbesondere ca. 40% verdünnt, beispielsweise um die Viskosität zu verringern.

Wichtig ist dabei, dass sich nach Verdünnung eine im wesentlichen homogene Lösung bildet.

Dies erfolgt bevorzugt erst relativ kurz vor dem Einsatz in der Herstellung des Hydrogels, beispielsweise nicht mehr als 24 Stunden vorher, bevorzugt nicht mehr als 20, besonders bevorzugt nicht mehr als 12, ganz besonders bevorzugt nicht mehr als 6 und insbesondere nicht mehr als 3 Stunden vorher.

Das Verdünnungsmittel G ist ausgewählt aus der Gruppe bestehend aus Wasser, ein Gemisch von Wasser mit einem oder mehreren in Wasser unbegrenzt löslichen organischen Lösemittel oder ein Gemisch von Wasser mit einem oder mehreren einfachen oder mehrfachfunktionellen Alkoholen, z.B. Methanol und Glycerin. Die Alkohole tragen bevorzugt 1, 2 oder 3 Hydroxygruppen und weisen bevorzugt zwischen 1 bis 10, insbesondere bis 4 C-Atome auf. Bevorzugt sind primäre und sekundäre Alkohole.

Bevorzugte Alkohole sind Methanol, Ethanol, Isopropanol, Ethylenglykol, 1,2-Propandiol oder 1 ,3-Propandiol.

d) Falls erforderlich kann das Reaktionsgemisch einer Entfärbung, beispielsweise durch Behandlung mit Aktivkohle oder Metalloxiden, wie z.B. Aluminiumoxid, Siliciumoxid, Magnesiumoxid, Zirkonoxid, Boroxid oder Gemischen davon, in Mengen von beispielsweise 0,1 - 50 Gew%, bevorzugt 0,5 bis 25 Gew%, besonders bevorzugt 1-10 Gew% bei Temperaturen von beispielsweise 10 bis 100 °C, bevorzugt 20 bis 80 °C und besonders bevorzugt 30 bis 60 °C unterworfen werden.

Dies kann durch Zugabe des pulver oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

Die Entfärbung des Reaktionsgemisches kann an beliebiger Stelle des Aufarbeitungsverfahrens erfolgen, beispielsweise auf der Stufe des rohen Reaktionsgemisches oder nach gegebenenfalls erfolgter Vorwäsche, Neutralisation, Wäsche oder Lösungsmittelentfernung.

Das Reaktionsgemisch kann weiterhin einer Vorwäsche e) und/oder einer Neutralisation f) und/oder einer Nachwäsche g) unterworfen werden, bevorzugt lediglich einer Neutralisation f). Gegebenenfalls können Neutralisation f) und Vorwäsche e) in der Reihenfolge auch vertauscht werden.

Aus der wässrigen Phase der Wäschen e) und g) und/oder Neutralisation f) kann enthaltene (Meth)acrylsäure und/oder Katalysator C durch Ansäuern und Extraktion mit einem Lösungsmittel zumindest teilweise wiedergewonnen und von Neuem eingesetzt werden.

Zur Vor- oder Nachwäsche e) oder g) wird das Reaktionsgemisch in einem Waschapparat mit einer Waschflüssigkeit, beispielsweise Wasser oder einer 5 - 30 Gew%-igen, bevorzugt 5 - 20, besonders bevorzugt 5 - 15 Gew%-igen Kochsalz-, Kaliumchlorid-, Ammoniumchlorid-, Natriumsulfat- oder Ammoniumsulfatlösung, bevorzugt Wasser oder Kochsalzlösung, behandelt.

Das Mengenverhältnis Reaktionsgemisch : Waschflüssigkeit beträgt in der Regel 1: 0,1 -1, bevorzugt 1 : 0,2 - 0,8, besonders bevorzugt 1 : 0,3 - 0,7.

Die Wäsche oder Neutralisation kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche oder Neutralisation im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Vorzugsweise werden Siebboden- oder gepackte beziehungsweise Füllkörperkolonnen, Rührbehälter oder Mixer-Settler-Apparate, sowie gepulste Kolonnen oder solche mit rotierenden Einbauten eingesetzt.

Die Vorwäsche e) wird bevorzugt dann eingesetzt, wenn Metallsalze, bevorzugt Kupfer oder Kupfersalze als Inhibitoren (mit)verwendet werden.

Eine Nachwäsche g) kann zur Entfernung von Base- oder Salzspuren aus dem in f) neutralisierten Reaktionsgemisch vorteilhaft sein.

Zur Neutralisation f) kann das gegebenenfalls vorgewaschene Reaktionsgemisch, das noch geringe Mengen an Katalysator und die Hauptmenge an überschüssiger (Meth)acrylsäure enthalten kann, mit einer 5 - 25, bevorzugt 5 - 20, besonders bevorzugt 5 - 15 Gew%igen wässrigen Lösung einer Base, wie beispielsweise Alkali- oder Erdalkalimetalloxide, -hydroxide, -carbonate oder -hydrogencarbonate, bevorzugt Natronlauge, Kalilauge, Natriumhydrogencarbonat, Natriumcarbonat, Kaliumhydrogencarbonat, Kalziumhydroxid, Kalkmilch, Ammoniak, Ammoniakwasser oder Kaliumcarbonat, der gegebenenfalls 5-15 Gew% Kochsalz, Kaliumchlorid, Ammoniumchlorid oder Ammoniumsulfat zugesetzt sein können, besonders bevorzugt mit Natronlauge oder Natronlauge-Kochsalz-Lösung, neutralisiert werden. Der Neutralisationsgrad beträgt bevorzugt 5 bis 60 Mol%, vorzugsweise 10 bis 40 Mol%, besonders bevorzugt 20 bis 30 Mol%, bezogen auf die Säuregruppen enthaltenden Monomere. Diese Neutralisation kann vor und/oder während der Polymerisation erfolgen, bevorzugt vor der Polymerisation.

Die Zugabe der Base erfolgt in einer Weise, dass die Temperatur im Apparat nicht über 60 °C ansteigt, bevorzugt zwischen 20 und 35 °C beträgt und der pH-Wert 4 - 13 beträgt. Die Abfuhr der Neutralisationswärme erfolgt vorzugsweise durch Kühlung des Behälters mit Hilfe von innenilegenden Kühlschlangen oder über eine Doppelwandkühlung.

Das Mengenverhältnis Reaktionsgemisch : Neutralisationsflüssigkeit beträgt in der Regel 1: 0,1 - 1, bevorzugt 1 : 0,2 - 0,8, besonders bevorzugt 1 : 0,3 - 0,7.

Hinsichtlich der Apparatur gilt das oben Gesagte.

h) Falls ein Lösungsmittel im Reaktionsgemisch enthalten ist, so kann dieses durch Destillation im wesentlichen entfernt werden. Bevorzugt wird gegebenenfalls enthaltenes Lösungsmittel nach Wäsche und/oder Neutralisation aus dem Reaktionsgemisch entfernt, falls gewünscht kann dieses aber auch vor der Wäsche beziehungsweise Neutralisation erfolgen.

Dazu wird das Reaktionsgemisch mit einer derartigen Menge an Lagerstabilisator, bevorzugt Hydrochinonmonomethylether versetzt, dass nach Abtrennung des Lösungsmittels 100 - 500, bevorzugt 200 - 500 und besonders bevorzugt 200 - 400 ppm davon im Zielester (Rückstand) enthalten sind.

Die destillative Abtrennung der Hauptmenge an Lösungsmittel erfolgt beispielsweise in einem Rührkessel mit Doppelwandheizung und/oder innenliegenden Heizschlangen unter vermindertem Druck, beispielsweise bei 20 - 700 mbar, bevorzugt 30 bis 500 und besonders bevorzugt 50-150 mbar und einer Temperatur von 40 - 80 °C.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 - 700 mbar, bevorzugt 30 bis 500 und besonders bevorzugt 50 - 150 mbar und einer Temperatur von 40 - 80 °C durch den Apparat geführt.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 - 1, bevorzugt 0,2 - 0,8 und besonders bevorzugt 0,3 - 0,7 $m^3/m^3h$, bezogen auf das Volumen des Reaktionsgemisches.

Der Restlösungsmittelgehalt im Rückstand beträgt nach der Destillation in der Regel unter 5 Gew%, bevorzugt 0,5 - 5 % und besonders bevorzugt 1 bis 3 Gew%.

Das abgetrennte Lösungsmittel wird kondensiert und bevorzugt wiederverwendet.

Falls erforderlich kann zusätzlich oder anstelle der Destillation eine Lösungsmittelstrippung i) durchgeführt werden.

Dazu wird der Zielester, der noch geringe Lösungsmittelmengen enthält, auf 50 - 90 °C, bevorzugt 80 - 90 °C erwärmt und die restlichen Lösungsmittelmengen mit einem geeigneten Gas in einer geeigneten Apparatur entfernt. Zur Unterstützung kann gegebenenfalls auch ein Vakuum angelegt werden.

Geeignete Apparaturen sind beispielsweise Kolonnen von an sich bekannter Bauart, die die üblichen Einbauten, z.B. Böden, Schüttungen oder gerichtete Packungen, bevorzugt Schüttungen aufweisen. Als Kolonneneinbauten kommen prinzipiell alle gängigen Einbauten in Betracht, beispielsweise Böden, Packungen und/oder Füllkörper. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten, bevorzugt.

Denkbar ist hier auch ein Fallfilm-, Dünnfilm- oder Wischfilmverdampfer, wie z.B. ein Luwa-, Rotafilm- oder Sambayverdampfer, der als Spritzschutz beispielsweise mit einem Demister ausgerüstet sein kann.

Geeignete Gase sind unter den Strippbedingungen inerte Gase, bevorzugt sauerstoffhaltige Gase, besonders bevorzugt Luft oder Gemische aus Luft und Stickstoff (Magerluft) oder Wasserdampf, insbesondere solche, die auf 50 bis 100 °C temperiert sind.

Die Strippgasmenge beträgt beispielsweise 5 - 20, besonders bevorzugt 10 - 20 und ganz besonders bevorzugt 10 bis 15 $m^3/m^3h$, bezogen auf das Volumen des Reaktionsgemisches.

Falls notwendig kann der Ester in einem beliebigen Stadium des Aufarbeitungsverfahrens, bevorzugt nach Wäsche/Neutralisation und gegebenenfalls erfolgter Lösungsmittelentfernung einer Filtration j) unterworfen werden, um

ausgefallene Spuren an Salzen sowie gegebenenfalls enthaltenem Entfärbungsmittel zu entfernen.

In einer denkbaren Ausführungsform wird die Veresterung a) von alkoxyliertem Glycerin mit der (Methacryl)säure in einem wie oben angeführten molaren Überschuss von mindestens 15:1 in Gegenwart mindestens eines Veresterungskatalysators C und mindestens eines Polymerisationsinhibitors D ohne ein mit Wasser ein Azeotrop bildendes Lösungsmittel durchgeführt.

Die im Überschuss eingesetzte (Methacryl)säure wird in einer bevorzugten Ausführungsform im wesentlichen nicht abgetrennt, d.h. es wird lediglich der Anteil an (Methacryl)säure aus dem Reaktionsgemisch entfernt, der durch die Flüchtigkeit bei der angelegten Temperatur bestimmt ist und es werden darüberhinaus keine Maßnahmen zur Abtrennung der Carbonsäure durchgeführt, wie beispielsweise destillative, rektifikative, extraktive, wie z.B. Wäschen, absorptive, wie z.B. Überleiten über Aktivkohle oder über Ionentauscher, und/oder chemische Schritte, wie z.B. Abfangen der Carbonsäure mit Epoxiden.

Bevorzugt wird die im Reaktionsgemisch enthaltene (Methacryl)säure zu nicht mehr als 75 Gew%, besonders bevorzugt zu nicht mehr als 50 Gew%, ganz besonders bevorzugt zu nicht mehr als 25 Gew%, insbesondere zu nicht mehr als 10 Gew% und speziell zu nicht mehr als 5 Gew% aus dem Reaktionsgemisch abgetrennt, bezogen auf die nach Reaktionsende im Reaktionsgemisch enthaltene (Methacryl)säure. In einer besonders bevorzugten Ausführungsform kann auf die Stufe b) verzichtet werden, so dass lediglich der Anteil an Reaktionswasser und (Methacryl)säure aus dem Reaktionsgemisch entfernt wird, der durch die Flüchtigkeit bei der angelegten Temperatur bestimmt ist. Dies kann bevorzugt durch im wesentlichen vollständige Kondensation verhindert werden.

Weiterhin verbleibt auch der eingesetzte Veresterungskatalysator C im wesentlichen im Reaktionsgemisch.

Das so erhältliche Reaktionsgemisch weist bevorzugt eine Säurezahl gem. DIN EN 3682 von mindestens 25 mg KOH/g Reaktionsgemisch auf, besonders bevorzugt von 25 bis 80 und ganz besonders bevorzugt von 25 bis 50 mg KOH/g auf.

Auf eine Vor- oder Nachwäsche e) oder g) wird bevorzugt verzichtet, lediglich ein Filtrationsschritt j) kann sinnvoll sein.

Anschließend kann das Reaktionsgemisch im Schritt c) verdünnt werden, in diesem Fall wird es bevorzugt innerhalb von 6 Stunden, besonders bevorzugt innerhalb von 3 Stunden zum Hydrogel umgesetzt. Bevorzugt kann es in einem Schritt f) neutralisiert werden.

Die Abfolge der Schritte c), j) und f) ist dabei beliebig.

Die Erfindung betrifft außerdem ein Stoffgemisch enthaltend

- mindestens einen Ester F, erhältlich nach einem der oben beschriebenen Veresterungsverfahren,
- (Methacryl)säure und
- Verdünnungsmittel G.

Als weitere Komponenten können enthalten sein

- Veresterungskatalysator C in protonierter oder unprotonierter Form,
- Polymerisationsinhibitor D sowie
- gegebenenfalls Lösungsmittel E, falls ein solches in der Versterung verwendet wurde.

Das Stoffgemisch kann gegebenenfalls neutralisiert sein und einen pH-Wert aufweisen, wie oben unter f) aufgeführt. Wenn das Stoffgemisch neutralisiert ist, so ist zumindest ein Teil der (Methacryl)säure in deren wasserlöslichen Alkalimetall-, Erdalkalimetall- oder Ammoniumsalze überführt.

Bevorzugtes Stoffgemisch enthält an

- Ester F im Stoffgemisch 0,1 bis 40 Gew.-%, besonders bevorzugt 0,5 bis 20, ganz besonders bevorzugt 1 bis 10, insbesondere 2 bis 5 und speziell 2 bis 4 Gew.-%,
- Monomer M 0,5 - 99,9 Gew%, besonders bevorzugt 0,5 - 50 Gew%, ganz besonders bevorzugt 1 - 25, insbesondere 2 -15 und speziell 3 bis 5 Gew%,
- Veresterungskatalysator C 0 - 10 Gew%, besonders bevorzugt 0,02 - 5, ganz besonders bevorzugt 0,05 - 2,5 Gew% und insbesondere 0,1 - 1 Gew%,
- Polymerisationsinhibitor D 0 - 5 Gew%, besonders bevorzugt 0,01 -1,0, ganz besonders bevorzugt 0,02 - 0,75, insbesondere 0,05 - 0,5 und speziell 0,075 - 0,25 Gew%,
- Lösungsmittel E 0 - 10 Gew%, besonders bevorzugt 0 - 5 Gew%, ganz besonders bevorzugt 0,05 - 1,5 Gew% und insbesondere 0,1 - 0,5 Gew%, mit der Maßgabe, dass die Summe immer 100 Gew% beträgt, sowie
- gegebenenfalls Verdünnungsmittel G ad 100 Gew%.

Die nach dem obigen Verfahren erhältlichen Reaktionsgemische und erfindungsgemäßen Stoffgemische können Verwendung finden

- als Radikalvernetzer von wasserabsorbierenden Hydrogelen,
- als Ausgangsstoff für die Herstellung von Polymerdispersionen,

- als Ausgangsstoff für die Herstellung von Polyacrylaten (außer Hydrogelen),
- als Lackrohstoff oder
- als Zementadditiv.

Zur Verwendung als Radikalvernetzer von wasserabsorbierenden Hydrogelen sind insbesondere solche erfindungsgemäßen Stoffgemische geeignet, die eine Wasserlöslichkeit (bei 25 °C in destilliertem Wasser) von mindestens 5 Gew%, bevorzugt mindestens 10 Gew%, besonders bevorzugt mindestens 20 Gew%, ganz besonders bevorzugt mindestens 30 Gew% und insbesondere von mindestens 50 Gew% aufweisen.

k) Das Reaktionsgemisch aus der Veresterung, inklusive deren Aufarbeitungsschritte, soweit diese durchlaufen werden, beispielsweise das Reaktionsgemisch aus f), bzw, wenn auf f) verzichtet wird; aus b), beziehungsweise, wenn auf b) verzichtet wird, das Reaktionsgemisch aus a), kann gegebenenfalls mit zusätzlichen monoethylenisch ungesättigten Verbindungen N versetzt werden, die keine Säuregruppen tragen, aber mit den hydrophilen Monomeren M copolymerisierbar sind, kann dann zur Herstellung von wasserabsorbierenden Hydrogelen in Gegenwart mindestens eines Radikalstarters K und gegebenenfalls mindestens einer Pfropfgrundlage L polymerisiert werden.

Vorteilhaft kann

l) das Reaktionsgemisch aus k) nachvernetzt werden.

Zur Herstellung k) dieser hydrophilen, hochquellfähigen Hydrogele geeignete hydrophile Monomere M sind beispielsweise polymerisationsfähige Säuren, wie Acrylsäure, Methacrylsäure, Ethacrylsäure, $\alpha$-Chloracrylsäure, Crotonsäure, Maleinsäure, Maleinsäureanhydrid, Vinylsulfonsäure, Vinylphosphonsäure, Maleinsäure einschließlich deren Anhydrid, Fumarsäure, Itaconsäure, Citraconsäure, Mesaconsäure, Glutaconsäure, Aconitsäure, Allylsulfonsäure, Sulfoethylacrylat, Sulfomethacrylat, Sulfopropylacrylat, Sulfopropylmethacrylat, 2-Hydroxy-3-acryloxypropylsulfonsäure, 2-Hydroxy-3-methacryl-oxypropylsulfonsäure, Allylphosphonsäure, Styrolsulfonsäure, 2-Acrylamido-2methylpropansulfonsäure, 2-Acrylamido-2-methylpropanphosphonsäure sowie deren Amide, Hydroxyalkylester und aminogruppen- oder ammoniumgruppenhaltige Ester und Amide. Die Monomeren können allein oder in Mischung untereinander eingesetzt werden. Des weiteren wasserlösliche N-Vinylamide oder auch Diallyldimethylammoniumchlorid. Bevorzugte hydrophile Monomere sind Verbindungen der Formel V

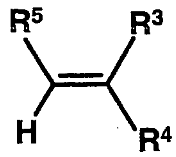

worin

$R^3$ Wasserstoff, Methyl oder Ethyl,

$R^4$ die Gruppe -$COOR^6$, eine Sulfonylgruppe oder Phosphonylgruppe, eine mit einem ($C_1$-$C_4$)-Alkylalkohol veresterte Phosphonylgruppe oder eine Gruppe der Formel VI

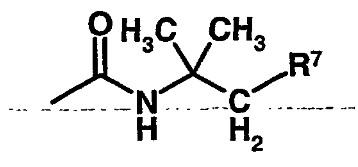

$R^5$ Wasserstoff, Methyl, Ethyl oder eine Carboxylgruppe,

$R^6$ Wasserstoff, Amino oder Hydroxy-($C_1$-$C_4$)-Alkyl und

$R^7$ eine Sulfonylgruppe, eine Phosphonylgruppe oder eine Carboxylgruppe bedeuten.

Beispiele für ($C_1$-$C_4$)- Alkylalkohol sind Methanol, Ethanol, n-Propanol oder n-Butanol.

Besonders bevorzugte hydrophile Monomere sind Acrylsäure und Methacrylsäure, insbesondere Acrylsäure.

Zur Optimierung von Eigenschaften kann es sinnvoll sein, zusätzliche monoethylenisch ungesättigte Verbindungen N einzusetzen, die keine Säuregruppen tragen, aber mit den säuregruppentragenden Monomeren copolymerisierbar sind. Hierzu gehören beispielsweise die Amide und Nitrile von monoethylenisch ungesättigten Carbonsäure, z. B. Acrylamid, Methacrylamid und N-Vinylformamid, N-Vinylacetamid, N-Methylvinylacetamid, Acrylnitril und Methacrylnitril. Weitere geeignete Verbindungen sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z. B. Ethylvinylether oder Butylvinylether, Ester von monoethylenisch ungesättigten $C_3$- bis $C_6$-Carbonsäuren, z. B. Ester aus einwertigen $C_1$- bis $C_{18}$-Alkoholen und Acrylsäure, Methacrylsäure oder Maleinsäure, Halbester von Maleinsäure, z. B. Maleinsäuremono-methylester, N-Vinyllactame wie N-Vinylpyrrolidon oder N-Vinylcaprolactam, Acrylsäure- und Methacrylsäureester von alkoxylierten einwertigen, gesättigten Alkoholen, z. B. von Alkoholen mit 10 bis 25 C-Atome, die mit 2 bis 200 Mol Ethylenoxid und/oder Propylenoxid pro Mol Alkohol umgesetzt worden sind, sowie Monoacrylsäureester und Monomethacrylsäuresster von Polyethylenglykol oder Polypropylenglykol, wobei die Molmassen ($M_n$) der Polyalkylenglykole beispielsweise bis zu 2000 betragen können. Weiterhin geeignete Monomere sind Styrol und alkylsubstituierte Styrole wie Ethylstyrol oder tert.-Butylstyrol.

Diese keine säuregruppentragenden Monomere können auch in Mischung mit anderen Monomeren eingesetzt werden, z. B. Mischungen aus Vinylacetat und 2-Hydroxyethylacrylat in beliebigem Verhältnis. Diese keine Säuregruppen tragenden Monomere werden der Reaktionsmischung in Mengen zwischen 0 und 50 Gew.-%, vorzugsweise kleiner 20 Gew.-% zugesetzt.

Bevorzugt bestehen die vernetzten (Co)Polymere aus Säuregruppen tragenden monoethylenisch ungesättigten Monomeren, die gegebenenfalls vor oder nach der Polymerisation in ihre Alkali-oder Ammoniumsalze überführt werden, und aus 0 - 40 Gew.-% bezogen auf ihr Gesamtgewicht keine säuregruppentragenden monoethylenisch ungesättigten Monomeren.

Die Herstellung, Testung und Verwendung (meth)acrylsäurehaltiger (Co)Polymere, Polyacrylsäuren und Superabsorbern ist vielfach vorbeschrieben und daher hinreichend bekannt, siehe z.B. "Modern Superabsorbent Polymer Technology", F.L. Buchholz and A.T. Graham, Wiley-VCH, 1998 oder in Markus Frank "Superabsorbents" in Ullmann's Handbuch der technischen Chemie Band 35 2003.

Bevorzugt sind solche Hydrogele, die durch vernetzende Polymerisation oder Copolymerisation von säuregruppentragenden monoethylenisch ungesättigten Monomeren M oder deren Salzen erhalten werden.

Die erhältlichen Polymere zeichnen sich durch einen verbesserten Verseifungsindex (VSI) aus.

Bei dem Verfahren zur Nachvernetzung wird das Ausgangspolymer mit einem Nachvernetzer behandelt und bevorzugt während oder nach dem Behandeln durch Temperaturerhöhung nachvernetzt und getrocknet, wobei der Vernetzer bevorzugt in einem inerten Lösemittel enthalten ist. Unter inerten Lösemittel werden solche verstanden, die bei der Reaktion weder mit dem Ausgangspolymer noch mit dem Nachvernetzer im wesentlichen reagieren. Bevorzugt sind solche Lösemittel, die zu mehr als 90%, bevorzugt mehr als 95%, besonders bevorzugt mehr als 99%, insbesondere zu mehr als 99,5% nicht chemisch mit dem Ausgangspolymer oder Nachvernetzer reagieren.

Bevorzugt zur Nachvernetzung l) und Trocknung m) ist dabei der Temperaturbereich zwischen 30 und 250°C, insbesondere 50 - 200°C, ganz besonders bevorzugt ist der Bereich zwischen 100 - 180°C. Die Aufbringung der Oberflächennachvernetzungslösung erfolgt bevorzugt durch Aufsprühen auf das Polymere in geeigneten Sprühmischern. Im Anschluss an das Aufsprühen wird das Polymerpulver thermisch getrocknet, wobei die Vernetzungsreaktion sowohl vor als auch während der Trocknung stattfinden kann. Bevorzugt ist das Aufsprühen einer Lösung des Vernetzers in Reaktionsmischern oder Misch- und Trocknungsanlagen wie beispielsweise Lödige-Mischer, BEPEX-Mischer, NAUTA-Mischer, SHUGGI-Mischer oder PROCESSALL. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner wie z.B. ein Hordentrockner, ein Drehrohrofen, oder eine beheizbare Schnecke. Es kann aber auch z.B. eine azeotrope Destillation als Trocknungsverfahren benutzt werden. Diebevorzugte Verweilzeit bei dieser Temperatur im Reaktionsmischer oder Trockner beträgt unter 60 min., besonders bevorzugt unter 30 min.

Bevorzugt sind obige Verfahren, wobei das Ausgangspolymere eine polymere Acrylsäure oder ein Polyacrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde.

Bevorzugt werden solche Verfahren in denen das Stoffgemisch enthaltend Radikalvernetzer, also den Ester F, und Verdünnungsmittel G in einem Verhältnis von 0,1 - 20 Gew.-%, insbesondere 0,5 - 10 Gew.-% bezogen auf die Masse des Ausgangspolymeren eingesetzt wird.

Bevorzugt werden solche Verfahren in denen der Radikalvernetzer in einer Dosierung von 0,01 - 5,0 Gew.%, bevorzugt 0,02 - 3,0 Gew.%, ganz besonders bevorzugt 0,03 - 2,5 Gew.%, insbesondere 0,05 - 1,0 und speziell 0,1 bis 0,75 Gew.% bezogen auf das Ausgangspolymer verwendet wird.

Gegenstand der Erfindung sind auch Polymere, hergestellt nach einem der oben genannten Verfahren und deren Verwendung in Hygieneartikeln, Verpackungsmaterialien und in Nonwovens, sowie die Verwendung von einem

oben genannten Stoffgemisch zur Herstellung von vernetzten oder durch Wärmebehandlung vernetzungsfähigen Polymeren, insbesondere in Lacken und Farben.

Die dabei einzusetzenden hydrophilen, hochquellfähigen Hydrogele (Ausgangspolymere) sind insbesondere Polymere aus (co)polymerisierten hydrophilen Monomeren M, Pfropf(co)polymere von einem oder mehreren hydrophilen Monomeren M auf eine geeignete Pfropfgrundlage L, vernetzte Cellulose- oder Stärkeether oder in wässrigen Flüssigkeiten quellbare Naturprodukte, wie beispielsweise Guarderivate. Diese Hydrogele sind dem Fachmann bekannt und beispielsweise beschrieben in US-4 286 082, DE-C-27 06 135, US-4 340 706, DE-C-37 13 601, DE-C-28 40 010, DE-A-43 44 548, DE-A40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18172, DE-A-30 28 043, DE-A-4418 881, EP-A-0 801 483, EP-A-0 455 985, EP-A-0 467 073, EP-A-0 312 952, EP-A-0 205 874, EP-A-0 499 774, DE-A 2612 846, DE-A-40 20 780 EP-A-0 20 5674, US-5 145 906, EP-A-0 530 438, EP-A-0 670 073, US4 057 521, US-4 062 817, US-4 525 527, US-4 295 987, US-5 011 892, US-4 076 663 oder US-4 931 497. Besonders geeignet sind auch hochquellfähige Hydrogele aus einem Herstellprozess wie in WO 01/38402 beschrieben, sowie anorganisch-organische hybride hochquellfähige Hydrogele wie in DE 198 54 575 beschrieben. Der Inhalt der vorstehend genannten Patentdokumente, insbesondere die nach den Verfahren hergestellten Hydrogele, sind ausdrücklich Bestandteil der vorliegenden Offenbarung.

Geeignete Pfropfgrundlagen L für hydrophile Hydrogele, die durch Pfropfcopolymerisation olefinisch ungesättigter Säuren erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Das wasserabsorbierende Polymer kann über radikalische Pfropfcopolymerisation von Acrylsäure oder Acrylat auf eine wasserlösliche Polymermatrix erhalten werden. Geeignete wasserlösliche Polymermatrices sind beispielsweise, aber nicht ausschliesslich, Alginate, Polyvinylalkohol, und Polysacharide wie etwa Stärke. Bei der Pfropfcopolymerisation im erfindungsgemäßen Sinne wird ein mehrfunktioneller ethylenisch ungesättigter Radikalvernetzer eingesetzt.

Das wasserabsorbierende Polymer kann ein organisch-anorganisches Hybridpolymer aus einer polymeren Acrylsäure oder einem Polyacrylat einerseits und einem Silikat, Aluminat, oder Alumosilikat andererseits sein. Insbesondere können polymere Acrylsäure oder Polyacrylat verwendet werden, die über radikalische Polymerisation erhalten wurden, und bei denen ein mehrfunktioneller ethylenisch ungesättigter Radikalvernetzer verwendet wurde und bei deren Herstellprozeß ein in Wasser lösliches Silikat oder lösliches Aluminat oder Gemische von beiden eingesetzt wurde.

Bevorzugte Hydrogele sind insbesondere Polyacrylate, Polymethacrylate sowie die in US-4 931 497, US-5 011 892 und US-5 041 496 beschriebene Pfropfpolymere. Ganz besonders bevorzugte Hydrogele sind die in WO 01/38402 beschriebenen Kneterpolymere und die in DE 198 545 75 beschriebenen hybriden organisch-anorganischen Hydrogele auf Basis von Polyacrylaten.

Die erfindungsgemäß hergestellten, als Radikalvernetzer in Hydrogelen verwendbaren Substanzen können allein oder in Kombination mit anderen Vernetzern, beispielsweise Innen- oder Oberflächenvernetzern verwendet werden, beispielsweise den folgenden:

Geeignete zusätzliche Vernetzer sind insbesondere Methylenbisacryl- bzw. -methacrylamid, Ester ungesättigter Mono- oder Polycarbonsäuren von Polyolen, wie Diacrylat oder Triacrylat, z. B. Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Besonders bevorzugt werden im erfindungsgemäßen Verfahren jedoch Hydrogele, die unter Verwendung von Polyallylethern als zusätzlichen Vernetzer und durch saure Homopolymerisation von Acrylsäure hergestellt werden. Geeignete Vernetzer sind Pentaerythritoltri- und - tetraallylether, Polyethylenglykoldiallylether, Monoethylenglykol-diallylether, Glyceroldi- und Triallylether, Polyallylether auf Basis Sorbitol, sowie ethoxylierte Varianten davon. Weiterhin besonders bevorzugte Vernetzer sind die Polyethylenglykol-diacrylate, ethoxylierte Derivate von Trimethylolpropantriacrylat z.B. Sartomer SR 9035, sowie ethoxylierte Derivate von Glycerindiacrylat und Glycerintriacrylat. Selbstverständlich können auch Gemische der obigen Vernetzer eingesetzt werden.

Ganz besonders bevorzugt sind solche Hydrogele, die mit einem erfindungsgemäß hergestellten Ester F als Radikalvernetzer hergestellt werden.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere enthalten (0,001 - 10 mol-%), ganz besonders bevorzugt sind jedoch Polymere, die über radikalische Polymerisation erhalten wurden und bei denen ein mehrfunktioneller ethylenisch ungesättigter Radikalvernetzer verwendet wurde.

Die hydrophilen, hochquellfähigen Hydrogele können durch an sich bekannte Polymerisationsverfahren hergestellt werden. Bevorzugt ist die Polymerisation in wässriger Lösung nach dem Verfahren der sogenannten Gelpolymerisation. Dabei werden wie oben angeführt verdünnte, bevorzugt wässrige, besonders bevorzugt 15 bis 50 Gew.-%

ige wässrige Lösungen eines oder mehrerer hydrophiler Monomerer und gegebenenfalls einer geeigneten Pfropfgrundlage L in Gegenwart eines Radikalinitiators bevorzugt ohne mechanische Durchmischung unter Ausnutzung des Trommsdorff-Norrish-Effektes (Makromol. Chem. 1, 169 (1947)), polymerisiert. Die Polymerisationsreaktion kann im Temperaturbereich zwischen 0°C und 150°C, vorzugsweise zwischen 10°C und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. Zur Auslösung der Polymerisation können energiereiche elektromagnetische Strahlen oder die üblichen chemischen Polymerisationsinitiatoren K herangezogen werden, z. B. organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie $(NH_4)_2S_2O_8$, $K_2S_2O_8$ oder $H_2O_2$.

Sie können gegebenenfalls in Kombination mit Reduktionsmitteln wie z.B. Ascorbinsäure, Natriumhydrogensulfit, und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie z. B. Mannichaddukte aus Sulfinsäuren, Aldehyden und Aminoverbindungen, wie sie in der DE-C-1 301 566 beschrieben sind, verwendet werden. Durch mehrstündiges Nachheizen der Polymerisatgele im Temperaturbereich 50° bis 130°C, vorzugsweise 70° bis 100°C, können die Qualitätseigenschaften der Polymerisate noch verbessert werden.

Die erhaltenen Gele werden zu 0- 100 mol%, bevorzugt zu 25-100 mol-%, und besonders bevorzugt zu 50 - 85 mol-% bezogen auf eingesetztes Monomer neutralisiert, wobei die üblichen Neutralisationsmittel verwendet werden können, bevorzugt Alkalimetallhydroxide, Alkalimetalloxide oder die entsprechenden Alkalimetallcarbonate, besonders bevorzugt jedoch Natriumhydroxid, Natriumcarbonat und Natriumhydrogencarbonat.

Üblicherweise wird die Neutralisation durch Einmischung des Neutralisationsmittels als wässrige Lösung oder bevorzugt auch als Feststoff erreicht. Das Gel wird hierzu mechanisch zerkleinert, z.B. mittels eines Fleischwolfes, und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt.

Die Polymerisation an sich kann aber auch nach jedem anderen der in der Literatur beschriebenen Verfahren durchgeführt werden. Insbesondere kann die Neutralisation der Acrylsäure auch vor der Polymerisation durchgeführt werden, wie oben im Schritt f) beschrieben. Die Polymerisation kann dann in einem dem Fachmann bekannten Bandreaktor oder einem Knetreaktor kontinuierlich oder auch diskontinuierlich durchgeführt werden. Bei Durchführung der Polymerisation in einem Bandreaktor ist die Initiation mittels elektromagnetischer Strahlung, bevorzugt mittels UV-Strahlung, oder alternativ die Initiation mit einem Redoxinitiatorsystem besonders bevorzugt. Ganz besonders bevorzugt ist auch die Kombination beider Initiationsmethoden: elektromagnetische Strahlung und chemisches Redoxinitiatorsystem simultan.

n) Das getrocknete Hydrogel kann hiernach gemahlen und gesiebt werden, wobei zur Mahlung üblicherweise Walzenstühle, Stiftmühlen oder Schwingmühlen eingesetzt werden können. Die bevorzugte Partikelgröße des gesiebten Hydrogels liegt vorzugsweise im Bereich 45 - 1000 μm, bevorzugt bei 45 - 850 μm, besonders bevorzugt bei 200 - 850 μm, und ganz besonders bevorzugt bei 300 - 850 μm. Ein anderer besonders bevorzugter Bereich liegt bei 150 - 850 μm, ganz besonders besonders bevorzugt bei 150 - 700 μm. In diesen Bereichen liegen bevorzugt 80 Gew.-% der Teilchen, insbesondere 90 Gew.-% der Teilchen. Die Größenverteilung kann mit etablierten Lasermethoden bestimmt werden.

Gegenstand der vorliegenden Erfindung sind weiterhin vernetzte Hydrogele, die mindestens ein hydrophiles Monomer M in einpolymerisierter Form enthalten und vernetzt sind mit einem Ester F von alkoxyliertem Glycerin mit (Methacryl)säure. Der Ester kann erfindungsgemäß oder auf eine im Stand der Technik bekannte Weise hergestellt werden, bevorzugt auf erfindungsgemäße Weise.

Als Ester F sind solche Verbindungen einsetzbar, wie sie vorstehend beschrieben sind.

Der CRC-Wert [g/g] der erfindungsgemäßen Hydrogel-formenden Polymere kann nach den in der Beschreibung angegebenen Methoden gemessen werden und liegt bevorzugt größer 15, insbesondere 16, 18, 20 ,22, 24, oder höher, besonders bevorzugt bei 25 insbesondere bei 26, 27, 28, 29, insbesondere bevorzugt bei 30, 31, 32, 33, 34, 35, 36, 37 oder höher.

Der AUL-0,7psi-Wert [g/g] der erfindungsgemäßen Hydrogel-formenden Polymere kann nach den in der Beschreibung angegebenen Methoden gemessen werden und liegt bevorzugt größer 8, insbesondere 9, 10, 11, 12, 13, 14 oder höher, besonders bevorzugt bei 15 insbesondere bei 16, 17, 18, 19, oder höher, insbesondere bevorzugt größer 20, insbesondere 21, 22, 23, 24, 25, 26, 27, 28, oder höher.

Der AUL-0,5psi-Wert [g/g] der erfindungsgemäßen Hydrogel-formenden Polymere kann nach den in der Beschreibung angegebenen Methoden gemessen werden und liegt bevorzugt größer 8, insbesondere 9, 10, 11, 12, 13, 14 oder höher, besonders bevorzugt bei 15 insbesondere bei 16, 17, 18, 19, oder höher, insbesondere bevorzugt größer 20, insbesondere 21, 22, 23, 24, 25, 26, 27, 28, oder höher.

Der Verseifungsindex VSI der erfindungsgemäßen Hydrogel-formenden Polymere kann nach den in der Beschreibung angegebenen Methoden gemessen werden und liegt bevorzugt kleiner 11, 10.5 oder 10, insbesondere 9.5, 9, oder 8.5 oder geringer, besonders bevorzugt kleiner 8, insbesondere 7.5, 7, 6.5, 6, 5.5 oder geringer, insbesondere bevorzugt kleiner 5, insbesondere 4.5, 4, 3.5 oder geringer.

Der Restvernetzergehalt der erfindungsgemäßen Hydrogel-formenden Polymere kann nach den in der Beschreibung angegebenen Methoden gemessen werden und liegt bevorzugt kleiner 10 ppm, insbesondere 9.5 ppm, 9 ppm, oder 8.5 ppm oder geringer, besonders bevorzugt kleiner 8 ppm, insbesondere 7.5 ppm, 7 ppm, 6.5 ppm, 6 ppm, 5.5 ppm oder geringer, insbesondere bevorzugt kleiner 5 ppm, insbesondere 4.5 ppm, 4 ppm, 3.5 ppm oder geringer.

Einsatz und Verwendung der erfindungsgemäßen Hydrogel-formenden Polymere

Die vorliegende Erfindung betrifft ferner die Verwendung der oben genannten Hydrogel-formenden Polymeren in Hygieneartikel, umfassend

(P) eine obere flüssigkeitsdurchlässige Abdeckung
(Q) eine untere flüssigkeitsundurchlässige Schicht
(R) einen zwischen (P) und (Q) befindlichen Kern, enthaltend
10-100 Gew.% des erfindungsgemäßem Hydrogel-formenden Polymer 0-90 Gew.-% hydrophiles Fasermaterial
bevorzugt 20-100 Gew.-% des erfindungsgemäßem Hydrogel-formenden Polymer, 0 - 80 Gew.-% hydrophiles Fasermaterial
mehr bevorzugt 30-100 Gew.-% des erfindungsgemäßem Hydrogel-formenden Polymer, 0-70 Gew.-% hydrophiles Fasermaterial
noch mehr bevorzugt 40-100 Gew.% des erfindungsgemäßem Hydrogel-formenden Polymer, 0-60 Gew.% hydrophiles Fasermaterial
besser bevorzugt 50-100 Gew.% des erfindungsgemäßem Hydrogel-formenden Polymer 0-50 Gew.-% hydrophiles Fasermaterial
besonders bevorzugt 60-100 Gew.-% des erfindungsgemäßem Hydrogel-formenden Polymer, 0 - 40 Gew.-% hydrophiles Fasermaterial
insbesondere bevorzugt 70-100 Gew.-% des erfindungsgemäßem Hydrogel-formenden Polymer, 0-30 Gew.-% hydrophiles Fasermaterial
außerordentlich bevorzugt 80-100 Gew.% des erfindungsgemäßem Hydrogel-formenden Polymer, 0-20 Gew.-% hydrophiles Fasermaterial
am meisten bevorzugt 90 - 100 Gew.-% des erfindungsgemäßem Hydrogel-formenden Polymer, 0 - 10 Gew.-% hydrophiles Fasermaterial
(S) gegebenenfalls eine sich unmittelbar oberhalb und unterhalb des Kerns (R) sich befindende Tissueschicht und
(T) gegebenenfalls eine zwischen (P) und (R) sich befindende Aufnahmeschicht.

Die Prozentangaben sind so zu verstehen, dass bei 10 - 100 Gew.%, 11, 12, 13, 14, 15, 16, 17, 18, 19 bis jeweils 100 Gew.-% an erfindungsgemäßem Hydrogel-formenden Polymer und alle dazwischenliegendnen %-Angaben (z.B. 12,2%) möglich sind und entsprechend hydrophiles Fasermaterial von 0 bis jeweils 89, 88, 87, 86, 85, 83, 82, 81 Gew.-% und dazwischen liegende Prozentangaben (z.B. 87,8%) möglich sind. Liegen weitere Materialien im Kern vor, so verringern sich entsprechend die Prozentwerte an Polymer und Faser. Das ananloge gilt für die bevorzugten Bereiche, so z.B. bei außerordentlich bevorzugt können 81, 82, 83, 84, 85, 86, 87, 88, 89 Gew.% für das erfindungsgemäßem Hydrogel-formenden Polymer und entsprechend 19, 18, 17, 16, 15, 14, 13, 12, 11 Gew-% des Fasermaterials vorliegen. So können im bevorzugten Bereich 20, 21, 22, 23, 24, 25, 26, 27, 28, 29 bis 100 Gew.-% erfindungsgemäßes Hydrogel-formendes Polymer, im mehr bevorzugten Bereich 30, 31, 32, 33, 34, 35, 36, 37, 38, 39 bis 100 Gew.-% erfindungsgemäßes Hydrogel-formendes Polymer, im noch mehr bevorzugten Bereich 40, 41, 42, 43, 44, 45, 46, 47, 48, 49 bis 100 Gew.-% erfindungsgemäßes Hydrogel-formendes Polymer, im besser bevorzugten Bereich 50, 51, 52, 53, 54, 55, 56, 57, 58, 59 bis 100 Gew.-% erfindungsgemäßes Hydrogel-formendes Polymer, im besonders bevorzugten Bereich 60, 61, 62, 63, 64, 65, 66, 67, 68, 69 bis 100 Gew.-% erfindungsgemäßes Hydrogel-formendes Polymer, im insbesonders bevorzugten Bereich 70, 71, 71, 72, 73, 74, 75, 76, 77, 78, 79 bis 100 Gew.-% erfindungsgemäßes Hydrogel-formendes Polymer und im am meisten bevorzugten Bereich 90, 91, 92, 93, 94, 95, 96, 97, 98, 99 oder 100 Gew.-% erfindungsgemäßes Hydrogel-formendes Polymer vorliegen.

Unter Hygieneartikel sind dabei sowohl Inkontinenzeinlagen und Inkontinenzhosen für Erwachsene als auch Windeln für Babys zu verstehen.

Bei der flüssigkeitsdurchlässigen Abdeckung (P) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355 A1, EP 102 388 3 A2.

Die flüssigkeitsundurchlässige Schicht (Q) besteht in der Regel aus einer Folie aus Polyethylen oder Polypropylen.

Der Kern (R) enthält neben dem erfindungsgemäßen Hydrogel-formendem Polymer hydrophiles Fasermaterial. Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 -200 $\mu$m, bevorzugt 10 - 100 $\mu$m. Darüberhinaus haben die Fasern eine Mindestlänge von 1 mm.

Der Aufbau und die Form von Windeln ist allgemein bekannt und beispielsweise in der WO 95 / 26 209 S. 66 Zeile 34 bis S. 69 Zeile 11, DE 196 04 601 A1, EP-A-0 316 518 und EP-A-0 202 127 beschrieben. Allgemein werden Windeln und andere Hygieneartikel auch in WO 00/65084, insbesondere auf Seiten 6-15, WO 00/65348, insbesondere auf Seiten 4 - 17, WO 00/35502, insbesondere Seiten 3-9, DE 19737434, WO 98/8439 beschrieben. Hygieneartikel für die Damenhygiene werden in folgenden Literaturstellen beschrieben. Die erfindungsgemäßen wässrige Flüssigkeiten absorbierende Hydrogel-formende Polymere können dort eingestzt werden. Literaturstellen Damenhygiene: WO 95/24173: Absorption Article for Controlling Odour, WO 91/11977: Body Fluid Odour Control, EP 389023: Absorbent Sanitary Articles, WO 94/25077: Odour Control Material, WO 97/01317: Absorbent Hygienic Article, WO 99/18905, EP 834297, US 5,762,644, US 5,895,381, WO 98/57609, WO 2000/065083, WO 2000/069485, WO 2000/069484, WO 2000/069481, US 6,123,693, EP 1104666, WO 2001/024755, WO 2001/000115, EP 105373, WO 2001/041692, EP 1074233. Tampons werden in folgenden Schriften beschrieben: WO 98/48753, WO 98/41179, WO 97/09022, WO 98/46182, WO 98/46181, WO 2001/043679, WO 2001/043680, WO 2000/061052, EP 1108408, WO 2001/033962, DE 200020662, WO 2001/001910, WO 2001/001908, WO 2001/001909, WO 2001/001906, WO 2001/001905, WO 2001/24729. Inkontinenzartikel werden in folgenden Schriften beschrieben: Disposable Absorbent Article for Incontinent Individuals: EP 311344 Beschreibung S. 3 - 9; Disposable Absorbent Article: EP 850623; Absorbent Article: WO 95/26207; Absorbent Article: EP 894502; Dry Laid Fibrous Structure: EP 850 616; WO 98/22063; WO 97/49365; EP 903134; EP 887060; EP 887059; EP 887058; EP 887057; EP 887056; EP 931530; WO 99/25284; WO 98/48753. Damenhygiene- und Inkontinenzartikel wird in folgenden Schriften beschrieben: Catamenial Device: WO 93/22998 Beschreibung S. 26 - 33; Absorbent Members for Body Fluids: WO 95/26209 Beschreibung S. 36 - 69; Disposable Absorbent Article: WO 98/20916 Beschreibung S. 13 - 24; Improved Composite Absorbent Structures: EP 306262 Beschreibung S. 3 - 14; Body Waste Absorbent Article: WO 99/45973. Diese Referenzen und die Referenzen dort, werden hiermit ausdrücklich in die Offenbarung der Erfindung einbezogen.

Die erfindungsgemäßen Hydrogel-formenden Polymere eignen sich hervorragend als Absorptionsmittel für Wasser und wässrige Flüssigkeiten, so dass sie vorteilhaft als Wasser zurückhaltendes Mittel im landwirtschaftlichen Gartenbau, als Filtrationshilfsmittel und besonders als saugfähige-Komponente in Hygieneartikein wie Windeln, Tampons oder Damenbinden eingesetzt werden können.

Einlagerung und Fixierung der erfindungsgemäßen hochquellfähigen Hydrogele

Zusätzlich zu den oben beschriebenen hochquellfähigen Hydrogelen liegen in der absorbierenden Zusammensetzung gemäß der vorliegenden Erfindung Kompositionen vor, welche die hochquellfähigen Hydrogele enthalten oder an denen sie fixiert sind. Jede Komposition ist geeignet, die die hochquellfähigen Hydrogele aufnehmen kann und die darüber hinaus in die Absorptionsschicht integriert werden kann. Eine Vielzahl derartiger Zusammensetzungen ist bereits bekannt und eingehend in der Literatur beschrieben. Eine Komposition zum Einbau der hochquellfähigen Hydrogele kann z. B. eine Fasermatrix sein, die aus einem Cellulosefasergemisch (airlaid web, wet laid web) oder aus synthetischen Polymerfasern (meltblown web, spunbonded web), oder aber aus einem Misch-Faserwerk aus Cellulosefasern und synthetischen Fasern besteht. Mögliche Fasermaterialien werden im sich anschließenden Kapitel detailliert beschrieben. Der Prozeß eines air-laid web ist beispielsweise geschildert in WO 98/28 478. Des weiteren können offenporige Schäume oder ähnliches zum Einbau hochquellfähiger Hydrogele dienen.

Alternativ kann eine derartige Komposition durch Fusion zweier Einzelschichten entstehen, wobei eine oder besser eine Vielzahl an Kammern gebildet werden, die die hochquellfähigen Hydrogele enthalten. Ein derartiges Kammersystem ist detailliert geschildert in EP 0 615 736 A1 S. 7 Zeile 26 ff.

In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstiges Gewebe, geschlossene oder offenporige Schäume, perforierte Filme, Elastomere oder Gewebe aus Fasermaterial zum Einsatz gelangen. Wenn die absorbierende Zusammensetzung aus einer Komposition von Schichten besteht, sollte das Schichtenmaterial eine Porenstruktur aufweisen, deren Porenabmessungen klein genug sind, um die hochquellfähigen Hydrogelpartikel zurückzuhalten. Obige Beispiele zur Komposition der absorbierenden Zusammensetzung schließen auch Laminate aus mindestens zwei Schichten mit ein, zwischen die die hochquellfähigen Hydrogele eingebaut und fixiert werden.

Generell besteht die Möglichkeit, Hydrogelpartikel innerhalb des Absorbent Cores zur Verbesserung der sog.

Dry- und Wet-Integrity zu fixieren. Unter Dry- und Wet-Integrity versteht man die Fähigkeit, hochquellfähige Hydrogele derart in die absorbierende Zusammensetzung einzubauen, dass sie äußeren Krafteinwirkungen sowohl im nassenals auch im trockenen Zustand standhalten und es nicht zu Verschiebungen oder Austritt von hochquellfähigem Polymer kommt. Unter Krafteinwirkungen sind vor allem mechanische Belastungen zu verstehen, wie sie im Bewegungsablauf beim Tragen des Hygieneartikels auftreten, oder aber die Gewichtsbelastung, unter der der Hygieneartikel vor allem im Inkontinenzfall steht. Zur Fixierung gibt es eine Vielzahl an Möglichkeiten, die dem Fachmann bekannt sind. Beispiele wie die Fixierung durch Wärmebehandlung, Zusatz von Adhäsiven, Thermoplasten, Bindermaterialien sind verzeichnet in WO 95/26 209 S. 37 Zeile 36 bis S. 41 Zeile 14. Besagte Passage ist somit Bestandteil dieser Erfindung. Methoden zur Erhöhung der Wet Strength finden sich auch in WO 2000/36216 A1.

Des weiteren kann die absorbierende Zusammensetzung aus einem Trägermaterial, wie z. B. einem Polymerfilm bestehen, auf dem die hochquellfähigen Hydrogelpartikel fixiert werden. Die Fixierung kann sowohl ein- als auch beidseitig vorgenommen werden. Das Trägermaterial kann wasserdurchlässig oder wasserundurchlässig sein.

In obige Kompositionen der absorbierenden Zusammensetzung werden die hochquellfähigen Hydrogele mit einem Gewichtsanteil von 10 bis 100 Gew%, bevorzugt 20-100 Gew.-%, mehr bevorzugt 30-100 Gew.-%, noch mehr bevorzugt 40-100 Gew.-%, besser bevorzugt 50 - 100 Gew.-%, besonders bevorzugt 60-100 Gew.-%, insbesondere bevorzugt 70 - 100 Gew.-%, außerordentlich bevorzugt 80 - 100 Gew.-% und am meisten bevorzugt 90-100 Gew.-% basierend auf dem Gesamtgewicht der Komposition und der hochquellfähigen Hydrogele eingebaut.

Fasermaterialien der absorbierenden Zusammensetzung

Dem Aufbau der vorliegenden erfindungsgemäßen absorbierenden Zusammensetzung liegen vielfältige Fasermaterialien zugrunde, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Einen detaillierten Überblick über Beispiele von Fasern, die in der vorliegenden Erfindung eingesetzt werden können, gibt Patent WO 95/26 209 S. 28 Zeile 9 bis S. 36 Zeile 8. Besagte Passage ist somit Bestandteil dieser Erfindung.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetraflourethylen, Polyvinylidenchlorid, Polyacrylverbindungen wie ORLON®, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie Polyethylenterephthalatfasern (DAC-RON® oder KODEL®), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abküh!en Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt zwischen 75°C und 175°C aufweisen. Bei diesen Temperaturen ist noch keine Schädigung der Cellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0,1 bis 100 Denier (Gramm pro 9 000 Meter) bevorzugt verwendet werden. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10 000 Meter). Die Form ist nicht besonders eingeschränkt und Beispiele schließen gewebeartige, schmale zylinderartige, geschnitten-/spaltgarnartige, stapelfaserartige und endlosfaserartige ein.

Die Fasern in der erfindungsgemäßen absorbierenden Zusammensetzung können hydrophil, hydrophob oder eine Kombination aus beiden sein. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner aus 90º ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90° ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die hochquellfähigen Hydrogele am stärksten hydrophil ist. Im Herstellungsprozeß wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zusammensetzung sind beispielsweise Cellulosefasern, modifizierte Cellulosefasern, Rayon, Polyesterfasern wie z. B. Polyethylenterephthalat (DACRON®), und hydrophiles Nylon (HYDROFIL®). Geeignete hydrophile Fasern können auch erhalten werden durch Hydrophilierung hydrophober Fasern, wie z.B. die Behandlung thermoplastischer Fasern, erhalten aus Polyolefinen (wie z. B. Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die hochquellfähigen Hydrogelpartikel werden in das beschriebene Fasermaterial eingebettet. Dies kann auf vielfältige Weise geschehen, indem man z. B. mit dem Hydrogelmaterial und den Fasern zusammen eine Absorptionsschicht in Form einer Matrix aufbaut, oder durch Einlagerung hochquellfähiger Hydrogele in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser: (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymene® 557 H, Hercoles, Inc. Wilmington Delaware, USA), Polyacrylamid- Überzüge (beschrieben in U.S. Patent 3,556,932 oder als Handelsprodukt der Marke Parez® 631 NC, American Cyanamid Co., Stamford, CT, USA), Melamin-Formaldehyd-Überzüge und Polyethylenimin-Überzüge mit ein.

Die chemische Versteifung von Cellulosefasern kann auch durch chemische Reaktion erfolgen. So kann z. B. die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind $C_2$-$C_8$ Dialdehyde, $C_2$-$C_8$ Monoaldehyde mit saurer Funktionalität, und insbesondere $C_2$-$C_9$ Polycarbonsäuren. Spezifische Substanzen aus dieser Reihe sind beispielswiese Glutaraldehyd, Glyoxal, Glyoxylsäure, Formaldehyd und Citronensäure. Diese Substanzen reagieren mit mindestens 2 Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Verteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt und in WO 91/11162, U.S. Patent 3,224,926, U.S. Patent 3,440,135, U.S. Patent 3,932,209, U.S. Patent 4,035,147, U.S. Patent 4,822,453, U.S. Patent 4,888,093, U.S. Patent 4,898,642 und U.S. Patent 5,137,537 beschrieben. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie z. B. Verschmelzen durch Wärmebehandlung, Zugabe von Schmelzklebern, Latexbindern usw. miteinander verbunden.

Herstellungsverfahren der absorbierenden Zusammensetzung

Die absorbierende Zusammensetzung setzt sich zusammen aus Kompositionen, welche hochquellfähige Hydrogele enthalten, und den hochquellfähigen Hydrogelen, die in besagten Kompositionen vorliegen oder daran fixiert sind.

Beispiele für Verfahren, mit denen man eine absorbierende Zusammensetzung erhält, die beispielsweise aus einem Trägermaterial bestehen, an den ein- oder beidseitig hochquellfähige Hydrogele fixiert sind, sind bekannt und von der Erfindung mit eingeschlossen, jedoch nicht limitiert darauf.

Beispiele für Verfahren, mit denen man eine absorbierende Zusammensetzng erhält, die beispielsweise aus

in ein Fasermaterial-Gemisch aus synthetischen Fasern (a) und Cellulosefasern (b) eingebetteten hochquellfähigen Hydrogelen (c) besteht, wobei das Mischungsverhältnis von (100 bis 0) synthetische Faser: (0 bis 100) Cellulosefaser variieren kann, schließen (1) ein Verfahren, bei dem (a), (b) und (c) gleichzeitig gemischt werden, (2) ein Verfahren, bei dem ein Gemisch aus (a) und (b) in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Die entsprechend erzeugte absorbierende Zusammensetzung kann optional einer Hitzebehandlung unterworfen werden, so dass eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich 60°C bis 230°C, bevorzugt zwischen 100°C und 200°C, besonders bevorzugt zwischen 100°C und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0,5 Sekunde bis 3 Minuten, bevorzugt 1 Sekunde bis 1 Minute.

Die absorbierende Zusammensetzung wird im allgemeinen beispielsweise mit einer für Flüssigkeit durchlässigen Deckschicht und einer für Flüssigkeit undurchlässigen Unterschicht versehen. Weiterhin werden Beinabschlüsse und Klebebänder angebracht und so der Hygieneartikel fertiggestellt. Die Materialien und Arten der durchlässigen Deckschicht und undurchlässigen Unterschicht, sowie der Beinabschlüsse und Klebebänder sind dem Fachmann bekannt und nicht besonders eingeschränkt. Beispiele hierfür finden sich in WO 95/26 209.

Der Vorteil der vorliegenden Erfindung beruht darin, dass die als Vernetzer verwendbaren Ester F nach ihrer Herstellung nicht aufgereinigt werden müssen, besonders dass nicht die (Meth)acrylsäure, bevorzugt Acrylsäure, abgetrennt werden muss, da diese in der Regel ein Monomer zur Herstellung der Hydrogele darstellt.

Experimenteller Teil

In dieser Schrift verwendete ppm- und Prozentangaben beziehen sich, falls nicht anders angegeben, auf Gewichtsprozente und -ppm.

Das erfindungsgemäße Verfahren wird durch das nachfolgende Beispiel näher erläutert.

Beispiele

Herstellung von Acrylat-Rohestern als Superabsorbervernetzer

Die Herstellung der Superabsorbervernetzer erfolgt in den Beispielen durch Veresterung von alkoxyliertem Glycerin mit Acrylsäure wobei die Abtrennung des Wassers in einer azeotropen Destillation erfolgt. Veresterungskatalysator ist in den Beispielen Schwefelsäure. Die Reaktanden werden zusammen mit einer Stabilisatormischung bestehend aus Hydrochinonmonomethylether, Triphenylphosphit und Hypophosphorige Säure in den Beispielen in Methylcyclohexan als Schleppmittel vorgelegt. Die Reaktionsmischung wird dann auf ca. 98°C erwärmt bis die azeotrope Destillation beginnt. Während der azeotropen Destillation steigt die Temperatur in der Reaktionsmischung an. Die abgetrennte Wassermenge wird bestimmt. Die Destillation wird abgebrochen, wenn mindestens die theoretische Wassermenge abgetrennt wurde. Anschließend wird das Schleppmittel in einer Vakuumdestillation entfernt. Das Produkt wird abgekühlt und als Vernetzer in der Superabsorberherstellung eingesetzt.

Umsatz und Ausbeute der Umsetzung wird nicht genau bestimmt, da das in der Veresterung abgetrennte Wasser auch Acrylsäure enthält und auch während der Vakuumdestillation des Schleppmittels Acrylsäure entfernt wird. Auch der Rohester enthält noch freie Acrylsäure, die zusammen mit dem Katalysator titriert wird (Säurezahl).

Alle Mengenangaben sind, soweit nicht anders angegeben, Gewichtsteile.

Herstellung des Esters

Säurezahlen wurden gem. DIN EN 3682 bestimmt.

Beispiel 1 Herstellung des alkoxylierten Glycerins

a) Glycerin-3 EO

77 g Glycerin werden mit 0,5 g KOH, 45 % in Wasser, in einem Autoklaven vorgelegt und zusammen bei 80°C

und reduziertem Druck (ca. 20 mbar) entwässert. Dann werden bei 145 bis 155°C 110 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei ca. 150 °C nachgerührt. Nach Spülen mit Inertgas und Abkühlen auf 60 °C wird der Katalysator durch Zugabe von Natriumpyrophosphat und anschließende Filtration abgetrennt.

b) Glycerin - 5 EO

77 g Glycerin werden mit 0,5 g KOH, 45 % in Wasser, in einem Autoklaven vorgelegt und zusammen bei 80°C und reduziertem Druck (ca. 20 mbar) entwässert. Dann werden bei 145 bis 155°C 184 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei ca. 150 °C nachgerührt. Nach Spülen mit Inertgas und Abkühlen auf 60 °C wird der Katalysator durch Zugabe von Natriumpyrophosphat und anschließende Filtration abgetrennt.

c) Glycerin - 3 PO - 2 EO (Blockpolymer)

77 g Glycerin werden mit 0,5 g KOH, 45 % in Wasser, in einem Autoklaven vorgelegt und zusammen bei 80°C und reduziertem Druck (ca. 20 mbar) entwässert. Dann werden bei 120 bis 130°C 146 g Propylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei 120 °C nachgerührt. Anschließend werden 74 g Ethylenoxid bei 145 bis 155°C über längere Zeit bei erhöhtem Druck zudosiert und ebenfalls abreagieren gelassen. Nach Spülen mit Inertgas und Abkühlen auf 60 °C wird der Katalysator durch Zugabe von Natriumpyrophosphat und anschließende Filtration abgetrennt.

d) Glycerin - 2 EO - 3 PO (Blockpolymer)

77 g Glycerin werden mit 0,5 g KOH, 45 % in Wasser, in einem Autoklaven vorgelegt und zusammen bei 80°C und reduziertem Druck (ca. 20 mbar) entwässert. Dann werden bei 145 bis 155 °C 74 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei ca. 150 °C nachgerührt. Anschließend werden 146 g Propylenoxid bei 120 bis 130 °C über längere Zeit bei erhöhtem Druck zudosiert und ebenfalls abreagieren gelassen. Nach Spülen mit Inertgas und Abkühlen auf 60 °C wird der Katalysator durch Zugabe von Natriumpyrophosphat und anschließende Filtration abgetrennt.

e) Glycerin - 2 EO - 3 PO (Mischpolymer)

77 g Glycerin werden mit 0,5 g KOH, 45 % in Wasser, in einem Autoklaven vorgelegt und zusammen bei 80°C und reduziertem Druck (ca. 20 mbar) entwässert. Dann werden bei 120 bis 130 °C 74 g Ethylenoxid sowie 146 g Propylenoxid zugegeben und die Temperatur wird langsam auf ca. 150 °C erhöht. Es wird unter erhöhtem Druck abreagieren gelassen. Die Reaktion ist beendet wenn keine Druckänderung mehr beobachtet wird. Es wird dann noch 30 min bei ca. 150 °C nachgerührt. Nach Spülen mit Inertgas und Abkühlen auf 60 °C wird der Katalysator durch Zugabe von Natriumpyrophosphat und anschließende Filtration abgetrennt.

Vergleichbeispiel a)

TMP - 3 EO

77 g Trimethylolpropan werden mit 0,5 g KOH, 45 % in Wasser, in einem Autoklaven vorgelegt und zusammen bei 80°C und reduziertem Druck (ca. 20 mbar) entwässert. Dann werden bei 145 bis 155°C 76 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei ca. 150 °C nachgerührt. Nach Spülen mit Inertgas und Abkühlen auf 60 °C wird der Katalysator durch Zugabe von Natriumpyrophosphat und anschließende Filtration abgetrennt.

Vergleichbeispiel b)

TMP-15 EO

77 g Trimethylolpropan werden mit 0,5 g KOH, 45 % in Wasser, in einem Autoklaven vorgelegt und zusammen

bei 80°C und reduziertem Druck (ca. 20 mbar) entwässert. Dann werden bei 145 bis 155°C 379 g Ethylenoxid zugegeben und bei dieser Temperatur unter erhöhtem Druck abreagieren gelassen. Die Reaktion ist beendet wenn keine Druckänderung mehr beobachtet wird. Es wird dann noch 30 min bei ca. 150 °C nachgerührt. Nach Spülen mit Inertgas und Abkühlen auf 60 °C wird der Katalysator durch Zugabe von Natriumpyrophosphat und anschließende Filtration abgetrennt.

Beispiel 2 Herstellung des Acrylsäureesters

a)

183 Teile ca. 3-fach ethoxyliertes Glycerin (gemäß Beispiel 1 a) wird mit 216 Teilen Acrylsäure und 5 Teilen Schwefelsäure in 345 Teilen Methylcyclohexan verestert. Als Hilfsstoffe werden 3 Teilen Hydrochinonmonomethylether, 1 Teil Triphenylphosphit und 1 Teil Hypophosphoriger Säure zugesetzt. Es werden 44 Teile Wasser abgetrennt bevor das Schleppmittel durch Vakuumdestillation entfernt wird. Das Produkt wird über K300-Filter gereinigt. Die Säurezahl wird bestimmt. Durch Zugabe von 96 Teilen Acrylsäure wird die Viskosität eingestellt. Die Viskosität des fast farblosen Produktes (Jodfarbzahl 0-1) beträgt ca. 330 mPas.

b)

255 Teile ca. 5-fach ethoxyliertes Glycerin (gemäß Beispiel 1 b) wird mit 216 Teilen Acrylsäure und 5 Teilen Schwefelsäure in 345 Teilen Methylcyclohexan verestert. Als Hilfsstoffe werden 3 Teilen Hydrochinonmonomethylether, 1 Teil Triphenylphosphit und 1 Teil Hypophosphoriger Säure zugesetzt. Es werden 44 Teile Wasser abgetrennt bevor das Schleppmittel durch Vakuumdestillation entfernt wird. Das Produkt wird über K300-Filter gereinigt. Die Säurezahl wird bestimmt. Durch Zugabe von 96 Teilen Acrylsäure wird die Viskosität eingestellt. Die Viskosität des fast farblosen Produktes (Jodfarbzahl 0-1) beträgt ca. 320 mPas.

c-e)

290 Teile ca. 2-fach ethoxyliertes 3-fach propoxyliertes Glycerin (gemäß Beispiel 1c-e) wird mit 216 Teilen Acrylsäure und 5 Teilen Schwefelsäure in 345 Teilen Methylcyclohexan verestert. Als Hilfsstoffe werden 3 Teilen Hydrochinonmonomethylether, 1 Teil Triphenylphosphit und 1 Teil Hypophosphoriger Säure zugesetzt. Es werden 44 Teile Wasser abgetrennt bevor das Schleppmittel durch Vakuumdestillation entfernt wird. Das Produkt wird über K300-Filter gereinigt. Die Säurezahl wird bestimmt. Durch Zugabe von 96 Teilen Acrylsäure wird die Viskosität eingestellt.

Herstellung von Hydrogelen

Zur Bestimmung der Güte der Oberflächenvernetzung kann das getrocknete Hydrogel mit folgenden Testmethoden untersucht werden.

Testmethoden

a) Zentrifugenretentionskapazität (CRC Centrifuge Retention Capacity)

Bei dieser Methode wird die freie Quellbarkeit des Hydrogels im Teebeutel bestimmt. Zur Bestimmung der CRC werden 0,2000 +/- 0,0050 g getrocknetes Hydrogel (Kornfraktion 106 - 850 μm) in einem 60 x 85 mm großen Teebeutel eingewogen, der anschließend verschweißt wird. Der Teebeutel wird für 30 Minuten in einen Überschuss von 0,9 Gew.%igen Kochsalzlösung gegeben (mindestens 0,83 l Kochsalz-Lösung / 1 g Polymerpulver). Anschließend wird der Teebeutel 3 Minuten lang bei 250 g zentrifugiert. Die Bestimmung der Flüssigkeitsmenge geschieht durch Auswägen des zentrifugierten Teebeutels.

b) Absorption unter Druck (AUL Absorbency Under Load) (0,7 psi)

Die Messzelle zur Bestimmung der AUL 0,7 psi stellt ein Plexiglas-Zylinder mit einem Innendurchmesser von 60 mm und einer Höhe von 50 mm dar, der an der Unterseite einen angeklebten Edelstahl-Siebboden mit einer Maschenweite von 36 μm besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 1345 g. Zur Durchführung der Bestimmung der AUL 0,7 psi wird das Gewicht des leeren Plexiglas-Zylinders und der Plastikplatte ermittelt und als Wo notiert. Dann werden 0,900 +/-

0,005 g Hydrogel-formendes Polymer (Korngrößenverteilung 150 - 800 μm) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm und einer Porosität 0 gelegt und soviel 0,9 Gew.%ige Natriumchloridlösung eingefüllt, dass die Flüssigkeitsoberfläche mit der Filterplattenoberfläche abschließt, ohne dass die Oberfläche der Filterplatte benetzt wird. Anschließend wird ein rundes Filterpapier mit einem Durchmesser von 90 mm und einer Porengröße < 20μm (S&S 589 Schwarzband von Schleicher & Schüll) auf die keramische Platte gelegt. Der Hydrogel-formendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\text{AUL 0,7 psi [g/g]} = [W_b - W_a] / [W_a - W_0]$$

Der AUL 0,5psi wird analog mit geringerem Druck gemessen.

c) Die Bestimmung des nach 16 h extrahierbare Anteils (Extract. 16h) erfolgte analog, wie in EP-A1 811 636, S. 13, Z. 1 bis Z. 19 beschrieben.

d) Methode zur Bestimmung von Restgehalten an Vernetzern in Hydrogelen

Zur Bestimmung des Gehaltes an unumgesetztem Restvernetzer extrahiert man diesen Restvernetzer zunächst mittels einer doppelten Extraktion aus dem getrockneten Hydrogel. Dazu wägt man 0,400 g trockenes Hydrogel und 40 g 0,9 Gew.%ige Kochsalzlösung in eine verschließbare und zentrifugierbare Ampulle ein. Hierzu fügt man 8 ml Dichlormethan, verschließt die Ampulle, und man lässt dann 60 min schütteln. Die Ampulle wird danach sofort 5 min lang bei 1500 Upm zentrifugiert, so dass die organische Phase klar von der wässrigen Phase getrennt ist.

In eine zweite Ampulle wägt man 50 μl Monoethylenglykol ein, und man fügt dazu ca. 5 - 6 ml des Dichlormethanextracts, das Gewicht des Extrakts wird auf 0,001 g genau erfasst. Bei 50-55 °C wird dann das Dichlormethan abgedampft und der Rückstand nach Abkühlen mit 2ml Methanol-Wasser-Gemisch (je 50 Volumenteile) aufgenommen. Es wird 10 min geschüttelt und dann über ein PTFE 0,45 μm-Filter filtriert.

Die so erhaltene Probe wird mittels Flüssigphasen-Chromatographie getrennt und massenspektrometrisch analysiert. Die Quantifizierung erfolgt gegen eine Verdünnungsreihe des selben eingesetzten Vernetzers.

Als Chromatographiesäule wird eine Zorbax Eclipse XDB C-8 (150 x 4.6 mm - 5 μm) und als Vorsäule eine Zorbax Eclipse XDB C-8 (12.5 x 4.6 mm - 5 μm) verwendet. Als Laufmittel wird ein Methanol/Wasser-Gemisch (75/25) eingesetzt.

Der Gradientenverlauf ist wie folgt:

| Zeit (min) | % Methanol | % Wasser |
|---|---|---|
| 0 | 75 | 25 |
| 3 | 75 | 25 |
| 4 | 98 | 2 |
| 8 | 98 | 2 |
| 9 | 75 | 25 |
| 14 | 75 | 25 |

Fluss ist 1 ml/min bei 1600 psi Druck.
Das Injektionsvolumen ist 20 μl.
Typische Analysenzeit ist 14 min für die Proben.

Die Detektion erfolgt massenspektrometrisch z.B. im Bereich 800 - 1300 m/z (full scan, positive). Das Gerät arbeitet mit APCI (Atmospheric Pressure Chemical Ionisation, positive Ionisierung). Zur Optimierung wird die Kapillartemperatur auf 180 °C, die APCI vaporizer Temperatur auf 450 °C, source current auf 5.0 μA, und der Gas Strom auf 80 ml/min eingestellt.

Die individuellen Einstellungen müssen für jeden Vernetzer speziell vorgenommen werden. Dazu ermittelt man

mittels einer geeigneten Kalibrierlösung des Vernetzers die später für die Auswertung relevanten charakteristischen Peaks. In der Regel wird der Hauptpeak ausgewählt.

Die Berechnung der Restvernetzerkonzentration ist dann wie folgt:

$$CONC_{Probe} = A_{Probe} \times CONC_{Std} \times VF / A_{Std}$$

| | |
|---|---|
| $CONC_{Probe}$: mg/kg | ist gesuchte Restvernetzerkonzentration im trockenen Hydrogel in |
| $CONC_{Std}$ : | ist gesuchte Restvernetzerkonzentration in der Kalibrierlösung in mg/kg |
| $A_{Probe}$ : | ist Peakfläche der Extraktprobe des getrockneten Hydrogels |
| $A_{Std}$ : | ist Peakfläche der Kalibrierlösung |

VF ist der Verdünnungsfaktor:

$$VF = M_{DCM} \times M_{Solv} / (M_{Probe} \times M_{Extract})$$

$M_{DCM}$ ist Einwaage Dichlormethan zur Extraktion

$M_{Probe}$ ist Einwaage trockenes Hydrogel

$M_{Solv}$ ist Einwaage Methanol-Wasser-Gemisch + Monoethylenglykol

$M_{Extract}$ ist Einwaage Dichlormethan-Extrakt

Mittels Kalibration (mehrere Punkte z.B. im Bereich 0 - 50 ppm) ist dabei sicher zu stellen, dass die Bestimmung im linearen Bereich ausgeführt wird.

e) Verseifungsindex VSI

Das zerkleinerte Gel wird dann auf zwei unterschiedlichen Wegen weiterbehandelt:

Aufarbeitungsmethode 1:

Das zerkleinerte Gel wird auf Blechen mit Siebböden homogen in dünner Schicht verteilt und dann 24 h bei 80 ºC im Vakuum getrocknet. Diese Trocknung ist sehr produktschonend und ist daher der optimale Vergleichsstandard.

Das getrocknete Hydrogel wird dann gemahlen und die Siebfraktion von 300 - 600 Mikrometern wird isoliert.

Aufarbeitungsmethode 2:

Das zerkleinerte Gel wird zunächst in einem verschlossenen Kunststoffbeutel bei 90 ºC für 24 h getempert. Dann wird es auf Blechen mit Siebböden homogen in dünner Schicht verteilt und für 24 h bei 80 °C im Vakuum getrocknet. Diese Trocknung simuliert die in typischen Produktionsanlagen auftretenden Trocknungsbedingungen, die üblicherweise die Trocknungsleistung und den Durchsatz wegen des damit verbundenen Qualitätsabfalls limitieren.

Das getrocknete Hydrogel wird gemahlen und die Siebfraktion von 300 - 600 Mikrometern wird isoliert.

Die nach den beiden Aufarbeitungsmethoden erhaltenen Hydrogele werden durch Bestimmung der Teebeutelkapazität (CRC) sowie des Gehalts an Extrahierbaren nach 16 h und hinsichtlich des Gehaltes an nicht abreagiertem Restvernetzer charakterisiert. Zusätzlich wird der Feuchtegehalt bestimmt und falls dieser über 1 Gew% beträgt, wird er rechnerisch bei der Ermittlung dieser Eigenschaften berücksichtigt. Typischerweise liegt der Feuchtegehalt bei ca. 5 Gew.%.

Aus den Messwerten bestimmt man dann den Verseifungsindex (VSI) des Vernetzers im Gel, der sich wie folgt berechnet:

$$VSI = 0{,}5 \times (CRC_2 - CRC_1) + 0{,}5 \times (Extrahierbare_2 - Extrahierbare_1)$$

Die tiefgestellten Indizes bezeichnen hier die Aufarbeitungsmethode 1 bzw. 2. Der Verseifungsindex ist also umso größer, je mehr durch die Betriebstrocknung die Teebeutelkapazität ansteigt und je mehr der Anteil der Extrahierbaren dabei ansteigt. Beide Beiträge werden gleich gewichtet.

Generell ist es vorteilhaft Vernetzer einzusetzen, deren Verseifungsindex möglichst klein ist. Der ideale Vernetzer besitzt einen VSI von Null. Der Einsatz solcher Vernetzer ermöglicht es die Leistung der Betriebstrockner ohne Qualitätseinbußen bis zum technisch erreichbaren Maximum zu erhöhen. Grund dafür ist, dass sich die während der Polymerisation eingestellte Vernetzung - und damit die Eigenschaften des Endproduktes - nicht mehr durch Hydrolyse bei der Trocknung verändert.

Beispiel 3 Herstellung von Superabsorber unter Verwendung des Acrylsäureesters aus Beispiel 2a und anderer Innenvernetzer

Beispiel a

In einer säurefesten Kunststoffwanne werden 305 g Acrylsäure und 3204 g einer 37,3 Gew.%-igen Natriumacrylatlösung in 1465 g destilliertem Wasser gelöst. Dazu werden 12,2 g Glycerin-3EO-Triacrylat als Vernetzer sowie 0,61 g V-50 (2,2'-Azobisamidinopropandihydrochlorid) und 3,05 g Natriumpersulfat als Initiatoren zugegeben. Die Initiatoren werden dabei vorteilhaft in einem Teil des Ansatzwassers vorgelöst. Der Ansatz wird einige Minuten gut gerührt.

Dann lässt man ca. 30 min Stickstoffgas durch die mit Kunststofffolie abgedeckte Lösung in der Wanne perlen, um den Sauerstoff zu entfernen sowie den Vernetzer homogen zu verteilen. Schließlich fügt man 0,244 g Wasserstoffperoxid gelöst in 5 g Wasser sowie 0,244 g Ascorbinsäure gelöst in 5 g Wasser hinzu. Die Starttemperatur zu Beginn der Reaktion soll 11 -13 ºC sein. Die Schichtdicke der Reaktionslösung beträgt ca. 6 cm. Nach wenigen Minuten startet die Reaktion, man lässt adiabatisch abreagieren und lässt die thermisch isolierte Wanne noch für nicht länger als 30 min stehen bevor das Gel aufgearbeitet wird.

Zur Aufarbeitung des Gels wird der Gelblock zunächst in Stücke zerbrochen und dann durch einen Fleischwolf mit 6 mm-Lochscheibe zerkleinert.

Das zerkleinerte Gel wird dann auf zwei unterschiedlichen Wegen weiterbehandelt:

Aufarbeitungsmethode 1:

Das zerkleinerte Gel wird auf Blechen mit Siebböden homogen in dünner Schicht verteilt und dann 24 h bei 80 ºC im Vakuum getrocknet. Diese Trocknung ist sehr produktschonend und ist daher der optimale Vergleichsstandard. Das getrocknete Hydrogel wird dann gemahlen und die Siebfraktion von 300 - 600 Mikrometern wird isoliert.

Aufarbeitungsmethode 2:

Das zerkleinerte Gel wird zunächst in einem verschlossenen Kunststoffbeutel bei 90 ºC für 24 h getempert. Dann wird es auf Blechen mit Siebböden homogen in dünner Schicht verteilt und für 24 h bei 80 ºC im Vakuum getrocknet. Diese Trocknung simuliert die in typischen Produktionsanlagen auftretenden Trocknungsbedingungen, die üblicherweise die Trocknungsleistung und den Durchsatz wegen des damit verbundenen Qualitätsabfalls limitieren.

Das getrocknete Hydrogel wird gemahlen und die Siebfraktion von 300 - 600 Mikrometern wird isoliert.

Analog zu Beispiel a werden folgende weitere Beispiele hergestellt:

Tab. 1

| Bsp. Nr. | Vernetzertyp | Einsatzmenge bez. Acrylsäuremonomer | Einsatz-menge in g |
|---|---|---|---|
| a | Glycerin - 3 EO - triacrylat | 1 Gew.% | 12,2 g |
| b | Glycerin - 5 EO - triacrylat | 1 Gew.% | 12,2 g |
| c | Glycerin - 3 PO - 2 EO - triacrylat Blockpolymer | 1 Gew.% | 12,2 g |
| d | Glycerin - 2 EO - 3 PO -triacrylat Blockpolymer | 1 Gew.% | 12,2 g |

Tabelle fortgesetzt

| Bsp. Nr. | Vernetzertyp | Einsatzmenge bez. Acrylsäuremonomer | Einsatz-menge in g |
|---|---|---|---|
| e | Glycerin - 3 PO - 2 EO - triacrylat Mischpolymer | 1 Gew.% | 12,2 g |
| f | TMP - 3 EO - triacrylat | 1 Gew.% | 12,2 g |
| g | TMP - 15 EO - triacrylat | 1 Gew.% | 12,2 g |

Die erzielten Eigenschaften dieser Hydrogele werden in Tab. 2 zusammengefassf:

| Bsp | | CRC 1 | Extrahierbare 16h 1 | Vernetzer Restgehalt 1 | CRC | 2 Extrahierbare 16h 2 | Vernetzer Restgehalt 2 | VSI |
|---|---|---|---|---|---|---|---|---|
| | | [g/g] | [Gew.%] | [ppm] | [g/g] | [Gew.%] | [ppm] | |
| a | Gly-3EO | 30,9 | 3,7 | 6 | 41,3 | 13,6 | < 5 | 10,2 |
| b | Gly-5EO | 30,6 | 3,3 | 8 | 42,5 | 13,6 | < 5 | 11,1 |
| c | Gly-3PO-2EO (Block) | 31,5 | 5,2 | 10 | 39,7 | 13,5 | < 5 | 8,3 |
| d d | Gly-2EO-3PO (Block) | 30,8 | 4,9 4,9 | 8 8 | 38,0 38,0 | 12,7 12,7 | < 5 < 5 | 7,5 7,5. |
| e e | Gly-3PO-2EO (stat.) | 31,2 31,2 | 3,9 3,9 | 7 | 41,0 41,0 | 13,0 13,0 | <5 < 5 | 9,59,5 |
| f | TMP-3E0 | 36,6 | 4,4 | 857 | 70,6 | 44,2 | 1302 | 36,9 |
| g | TMP-15E0 | 29,7 | 2,8 | 51 | 43,1 | 12,6 | 20 | 11,6 |

Nachvernetzung:

Das trockene normale Grundpolymerpulver wird mit einer Lösung aus 0,06 Gew. % Ethylenglykoldiglycidylether (Firma Nagase, Japan), 3,43 Gew.% Wasser und 1,47 Gew.% Propandiol-1,2 jeweils bezogen auf eingesetztes Polymerunter Rühren homogen besprüht.

Das feuchte Pulver wird dann im Trockenschrank bei 150° C für 60 min getempert. Danach wird nochmals bei 850 Mikrometer gesiebt um Agglomerate zu entfernen. Die Eigenschaften dieses nachvernetzten Polymers werden bestimmt.

## Patentansprüche

1. Ester F der Formel Ia

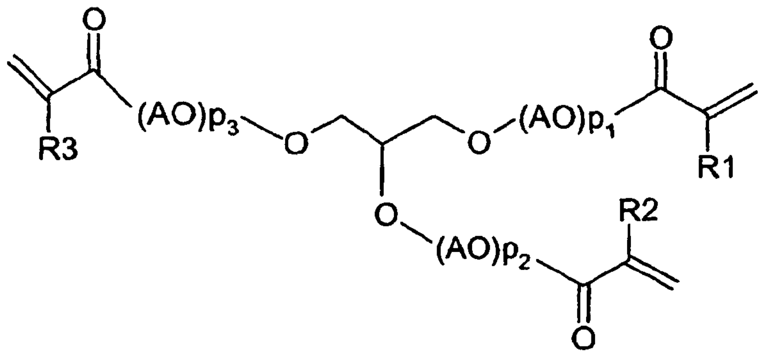

mit AO bedeutet für jedes AO unabhängig voneinander EO oder PO,
wobei EO bedeutet O-CH2-CH2-,
PO bedeutet unabhängig voneinander O-CH2-CH(CH3)- oder O-CH(CH3)-CH2-
p1 + p2 + p3 ist 3, 4 oder 5,
R1, R2, R3 unabhängig voneinander H oder CH3,
wobei mindestens ein AO PO und mindestens ein weiteres AO EO bedeutet.

2. Ester F der Formel Ib

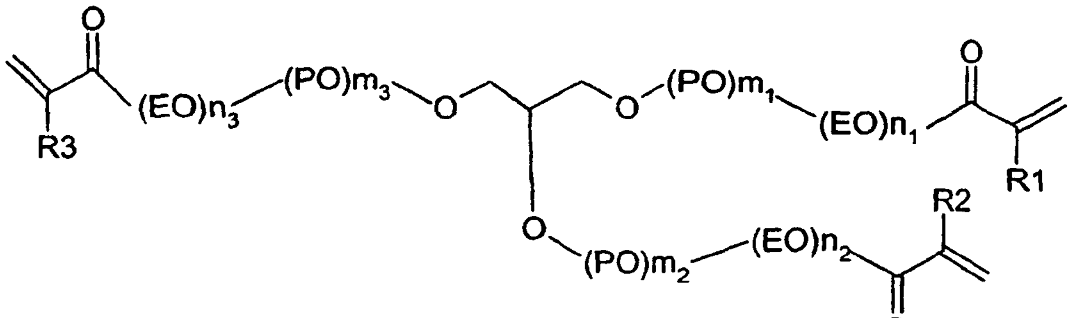

mit EO bedeutet O-CH2-CH2-,
PO bedeutet unabhängig voneinander O-CH2-CH(CH3)- oder O-CH(CH3)-CH2-
m1 + m2 + m3 + n1 + n2 + n3 ist 3, 4 oder 5,
m1 + m2 + m3 ist 1, 2, 3, oder 4,
R1, R2, R3 unabhängig voneinander H oder CH3,
wobei mindestens ein AO PO und mindestens ein weiteres AE EO bedeutet.

3. Ester F der Formel Ic

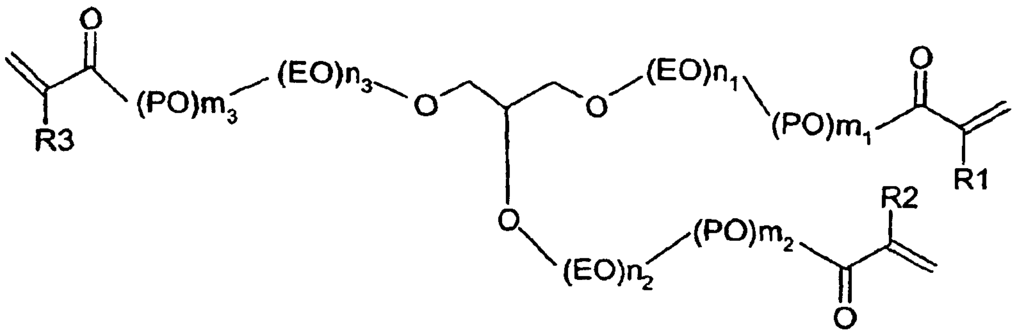

mit EO bedeutet O-CH2-CH2-
PO bedeutet unabhängig voneinander O-CH2-CH(CH3)- oder O-CH(CH3)-CH2-
m1 + m2 + m3 + n1 + n2 + n3 ist 3, 4 oder 5,
m1 + m2 + m3 ist 1, 2, 3, oder 4,
R1, R2, R3 unabhängig voneinander H oder CH3,
wobei mindestens ein AO PO und mindestens ein weiteres AE EO bedeutet.

4. Ester F gemäß einem der Ansprüche 1 bis 3, wobei m1 + m2 + m3 + n1 + n2 + n3 oder p1 + p2 + p3 gleich 3 oder 5 ist.

5. Ester F gemäß einem der Ansprüche 1 bis 4, wobei insgesamt 3 PO vorliegen.

6. Ester F gemäß einem der Ansprüche 1 bis 5, wobei in jeder der 3 Alkoxyketten des Glycerins mindestens 1 PO vorliegt.

7. Verfahren zur Herstellung eines Esters F gemäß einem der Ansprüche 1 bis 6 von alkoxyliertem Glycerin der Formel IIa, IIb oder IIc

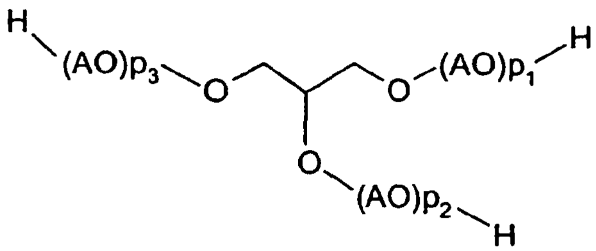

IIa,

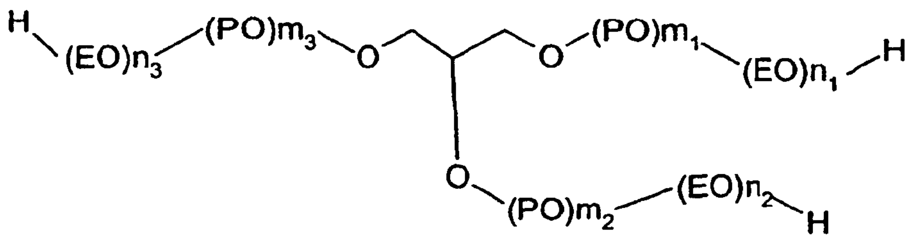

IIb,

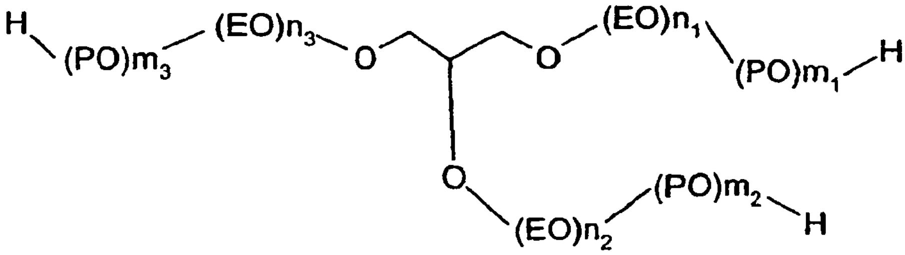

IIc,

wobei AO, EO, PO, n1, n2, n3, m1, m2, m3, p1, p2, p3 die Bedeutung besitzen wie in einem der Ansprüche 1 bis 6, mit (Meth)acrylsäure, umfassend die Schritte

a) Umsetzung von alkoxyliertem Glycerin mit (Meth)acrylsäure in Anwesenheit mindestens eines Veresterungskatalysators C und mindestens eines Polymerisationsinhibitors D sowie gegebenenfalls eines mit Wasser ein Azeotrop bildenden Lösungsmittels E unter Bildung eines Esters F,
b) gegebenenfalls Entfernen zumindest eines Teils des in a) entstehenden Wassers aus dem Reaktionsgemisch, wobei b) während und/oder nach a) erfolgen kann,
f) gegebenenfalls Neutralisation des Reaktionsgemischs,
h) falls ein Lösungsmittel E eingesetzt wurde gegebenenfalls Entfernen dieses Lösungsmittels durch Destillation und/oder
i) Strippen mit einem unter den Reaktionsbedingungen inerten Gas.

**8.** Verfahren nach Anspruch 7, **dadurch gekennzeichnet, dass**

- der molare Überschuss (Meth)acrylsäure zum alkoxyliertem Glycerin mindestens 3,15:1 beträgt und
- die in dem nach dem letzten Schritt erhaltenen Reaktionsgemisch enthaltene, gegebenenfalls neutralisierte (Meth)acrytsäure im wesentlichen im Reaktionsgemisch verbleibt.

**9.** Verfahren zur Herstellung eines Esters F gemäß einem der Ansprüche 1 bis 6 von alkoxyliertem Glycerin der Formel IIa, IIb oder IIc

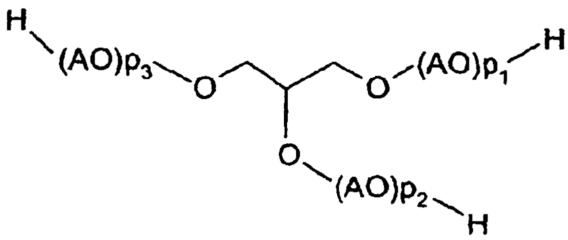

IIa,

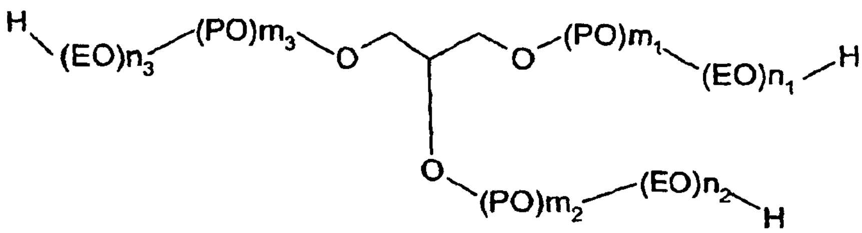

IIb,

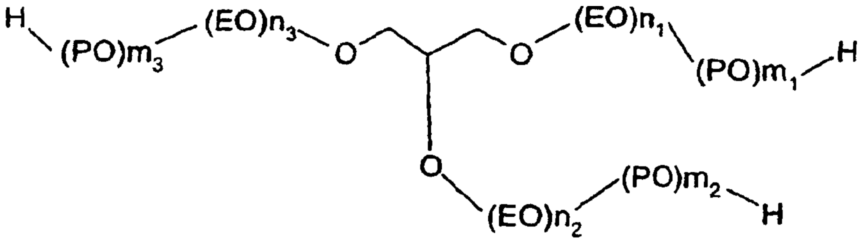

IIc,

wobei AO bedeutet für jedes AO unabhängig voneinander EO oder PO,
wobei EO bedeutet O-CH2-CH2-,
PO bedeutet unabhängig voneinander O-CH2-CH(CH3)- oder O-CH(CH3)-CH2-
p1 + p2 + p3 ist 3, 4 oder 5,
R1, R2, R3 unabhängig voneinander H oder CH3,
mit (Meth)acrylsäure, umfassend die Schritte

a) Umsetzung von alkoxyliertem Glycerin mit (Meth)acrylsäure in Anwesenheit mindestens eines Veresterungskatalysators C und mindestens eines Polymerisationsinhibitors D sowie gegebenenfalls eines mit Wasser ein Azeotrop bildenden Lösungsmittels E unter Bildung eines Esters F,
b) gegebenenfalls Entfernen zumindest eines Teils des in a) entstehenden Wassers aus dem Reaktionsgemisch, wobei b) während und/oder nach a) erfolgen kann,
f) gegebenenfalls Neutralisation des Reaktionsgemischs,
h) falls ein Lösungsmittel E eingesetzt wurde gegebenenfalls Entfernen dieses Lösungsmittels durch Destillation und/oder
i) Strippen mit einem unter den Reaktionsbedingungen inerten Gas,

**dadurch gekennzeichnet, dass**

- der molare Überschuss (Meth)acrylsäure zum alkoxyliertem Glycerin mindestens 3,15:1 beträgt und
- die in dem nach dem letzten Schritt erhaltenen Reaktionsgemisch enthaltene, gegebenenfalls neutralisierte (Meth)acrylsäure im wesentlichen im Reaktionsgemisch verbleibt.

**10.** Verfahren nach einem der Ansprüche 7 bis 9, **dadurch gekennzeichnet, dass** die (Meth)acrylsäure aus dem nach dem letzten Schritt erhaltenen, Ester F enthaltenden Reaktionsgemisch zu nicht mehr als 75 Gew.-% abgetrennt wird.

**11.** Verfahren nach einem der Ansprüche 7 bis 10, **dadurch gekennzeichnet, dass** das nach dem letzten Schritt erhaltene, Ester F enthaltende Reaktionsgemisch eine Säurezahl gem. DIN EN 3682 von mindestens 25 mg KOH/g aufweist.

**12.** Verfahren nach einem der Ansprüche 7 bis 11, **dadurch gekennzeichnet, dass** das nach dem letzten Schritt erhaltene, Ester F enthaltende Reaktionsgemisch einen Gehalt an (Meth)acrylsäure von mindestens 0,5 Gew.-% aufweist.

**13.** Verfahren nach einem der Ansprüche 7 bis 12, **dadurch gekennzeichnet, dass** in der Umsetzung a) das molare Verhältnis der (Meth)acrylsäure zum alkoxyliertem Glycerin mindestens 15:1 beträgt.

**14.** Verfahren zur Herstellung eines vernetzten Hydrogels, umfassend die Schritte

k) Polymerisieren eines Esters F gemäß einem der Ansprüche 1 bis 6, oder einem Ester F der Formel Ia

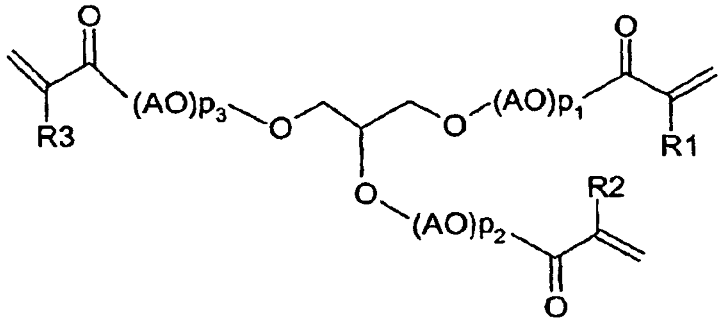

mit AO bedeutet für jedes AO unabhängig voneinander EO oder PO,
wobei EO bedeutet O-CH2-CH2-,
PO bedeutet unabhängig voneinander O-CH2-CH(CH3)- oder O-CH(CH3)-CH2-
p1 + p2 + p3 ist 3, 4 oder 5,
R1, R2, R3 unabhängig voneinander H oder CH3,
mit (Meth)acrylsäure, mit gegebenenfalls zusätzlichen monoethylenisch ungesättigten Verbindungen N, sowie gegebenenfalls mindestens einem weiteren copolymerisierbaren hydrophilen Monomer M in Gegenwart mindestens eines Radikalstarters K und gegebenenfalls mindestens einer Pfropfgrundlage L,
l) gegebenenfalls Nachvernetzung des aus k) erhaltenen Reaktionsgemisches,
m) Trocknung des aus k) oder l) erhaltenen Reaktionsgemisches und
n) gegebenenfalls Mahlen und/oder Sieben des aus k), l) oder m) erhaltenen Reaktionsgemisches.

**15.** Verfahren nach Anspruch 14, wobei beim Ester F AO EO bedeutet.

**16.** Verfahren zur Herstellung eines vernetzten Hydrogels, umfassend die Schritte a) bis i) gemäß einem der Ansprüche 7 bis 15 und zusätzlich

k) Polymerisieren des Reaktionsgemischs aus einer der Stufen a) bis i), soweit durchlaufen, mit gegebenenfalls zusätzlichen monoethylenisch ungesättigten Verbindungen N, sowie gegebenenfalls mindestens einem weiteren copolymerisierbaren hydrophilen Monomer M in Gegenwart mindestens eines Radikalstarters K und gegebenenfalls mindestens einer Pfropfgrundlage L,
1) gegebenenfalls Nachvernetzung des aus k) erhaltenen Reaktionsgemisches,
m) Trocknung des aus k) oder 1) erhaltenen Reaktionsgemisches und
n) gegebenenfalls Mahlen und/oder Sieben des aus k), 1) oder m) erhaltenen Reaktionsgemisches.

**17.** Polymer, erhältlich nach einem Verfahren gemäß einem der Ansprüche 14 bis 16.

**18.** Vernetztes Hydrogel, enthaltend mindestens ein hydrophiles Monomer M in einpolymerisierter Form, vernetzt mit einem Ester F gemäß einem der Ansprüche 1 bis 6 oder einem Ester F der Formel Ia

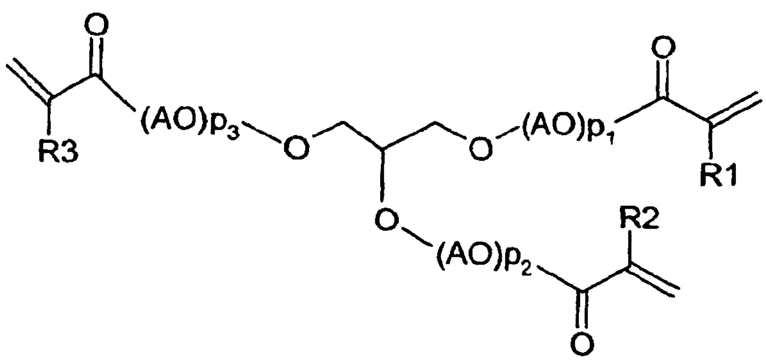

mit AO bedeutet für jedes AO unabhängig voneinander EO oder PO,

wobei EO bedeutet O-CH2-CH2-,
PO bedeutet unabhängig voneinander O-CH2-CH(CH3)- oder O-CH(CH3)-CH2-
p1 + p2 + p3 ist 3, 4 oder 5,
R1, R2, R3 unabhängig voneinander H oder CH3.

**19.** Vernetztes Hydrogel, enthaltend mindestens ein hydrophiles Monomer M in einpolymerisierter Form, vernetzt mit einem Ester F enthaltenden Reaktionsgemisch, wie es erhältlich nach einem Verfahren der Ansprüche 7 bis 13 ist.

**20.** Verwendung eines Polymers gemäß einem der Ansprüche 17 bis 19 in Hygieneartikeln. Verpackungsmaterialien und in Nonwovens.

**21.** Stoffgemisch, enthaltend

- 0,1 bis 40 Gew.- % mindestens eines Esters F gemäß einem der Ansprüche 1 bis 6 oder einem Ester F der Formel la

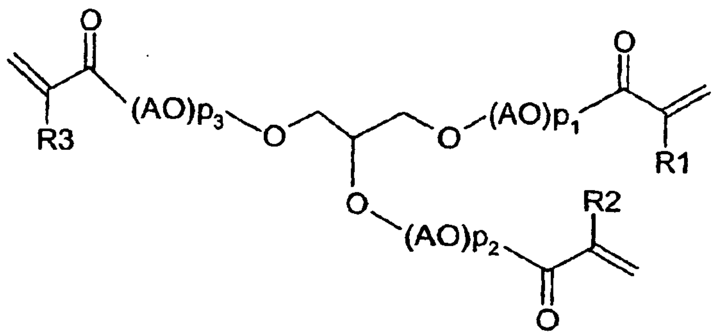

mit AO bedeutet für jedes AO unabhängig voneinander EO oder PO,

wobei EO bedeutet O-CH2-CH2-,
PO bedeutet unabhängig voneinander O-CH2-CH(CH3)- oder O-CH(CH3)-CH2-
p1 + p2 + p3 ist 3, 4 oder 5,
R1, R2, R3 unabhängig voneinander H oder CH3,
und (Meth)acrylsäure,

- 0,5 - 99,9 Gew.-% mindestens eines hydrophilen Monomers M,
- 0 - 10 Gew.-% mindestens eines Veresterungskatalysators C,
- 0 - 5 Gew.-% mindestens einen Polymerisationsinhibitors D und
- 0 - 10 Gew.-% eines Lösungsmittels E,

mit der Maßgabe, dass die Summe immer 100 Gew.-% beträgt.

**22.** Stoffgemisch gemäß Anspruch 21, enthaltend zusätzlich

- Verdünnungsmittel G ad 100 Gew.-%.

**23.** Vernetztes Hydrogel, erhältlich aus einem Stoffgemisch gemäß Anspruch 21 oder 22 und zusätzlich

l) gegebenenfalls Nachvernetzung des erhaltenen Reaktionsgemisches,
m) Trocknung des direkt erhaltenen oder aus l) erhaltenen Reaktionsgemisches und
n) gegebenenfalls Mahlen und/oder Sieben des direkt erhaltenen oder aus l) oder m) erhaltenen Reaktionsgemisches.

**24.** Verwendung eines Reaktionsgemisches erhältlich nach einem der Ansprüche 7 bis 13 oder eines Stoffgemisches nach Anspruch 21 oder 22

- als Radikalvernetzer von wasserabsorbierenden Hydrogelen,
- als Ausgangsstoff für die Herstellung von Polymerdispersionen,
- als Ausgangsstoff für die Herstellung von Polyacrylaten,
- als Lackrohstoff oder
- als Zementadditiv.

**25.** Vernetztes Hydrogel mit einem Verseifungsindex kleiner 10, bevorzugt kleiner 8, insbesondere kleiner 5.

**26.** Vernetztes Hydrogel gemäß einem der Ansprüche 17, 18, 19 oder 23 mit einem Verseifungsindex kleiner 11, bevorzugt kleiner 10, besonders bevorzugt kleiner 8, insbesondere kleiner 5.

**27.** Vernetztes Hydrogel gemäß einem der Ansprüche 17, 18, 19, 23, 25 oder 26 mit einem Restvernetzergehalt kleiner 10 ppm, bevorzugt kleiner 8 ppm, besonders bevorzugt kleiner 5 ppm.

**28.** Verwendung eines Esters F gemäß einem der Ansprüche 1 bis 6 oder einem Ester der Formel la

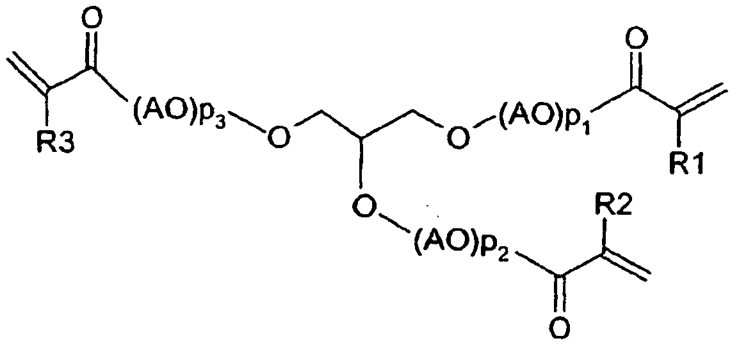

mit AO bedeutet für jedes AO unabhängig voneinander EO oder PO,
wobei EO bedeutet O-CH2-CH2-,
PO bedeutet unabhängig voneinander O-CH2-CH(CH3)- oder O-CH(CH3)-CH2-
p1 + p2 + p3 ist 3, 4 oder 5,
R1, R2, R3 unabhängig voneinander H oder CH3,
zur Herstellung von wässrigen Flüssigkeiten absorbierende Hydrogel-formende Polymere.

## Claims

**1.** An ester F of the formula la

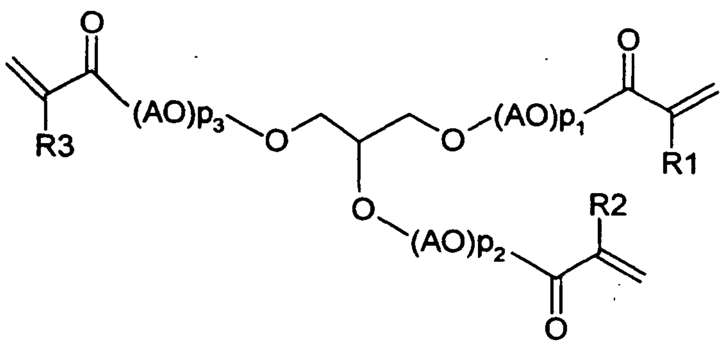

where AO is for each AO independently EO or PO,
where EO is O-CH2-CH2-,
PO is at each instance independently O-CH2-CH(CH3)- or O-CH(CH3)-CH2-
p1 + p2 + p3 is 3, 4 or 5,

R1, R2 and R3 are independently H or CH3,
wherein at least one AO is PO and at least one further AO is EO.

**2.** An ester F of the formula Ib

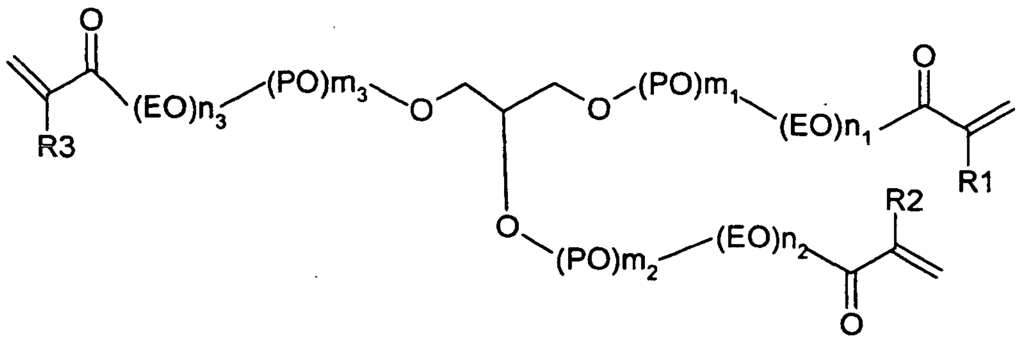

where EO is O-CH2-CH2-
PO is at each instance independently O-CH2-CH(CH3)- or O-CH(CH3)-CH2-
m1 + m2 + m3 + n1 + n2 + n3 is 3, 4 or 5.
m1 + m2 + m3 is 1, 2, 3, or 4,
R1, R2, R3 are independently H or CH3, wherein at least one AO is PO and at least one further AO is EO.

**3.** An ester F of the formula Ic

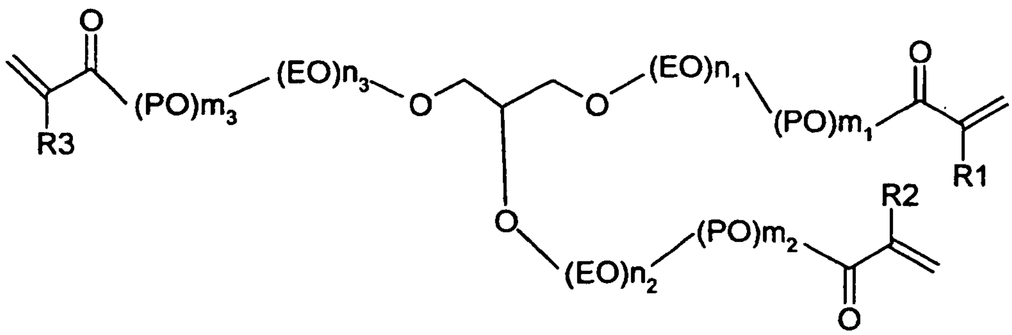

where EO is O-CH2-CH2-
PO is at each instance independently O-CH2-CH(CH3)- or O-CH(CH3)-CH2-
m1 + m2 + m3 + n1 + n2 + n3 is 3, 4 or 5,
m1 + m2 + m3 is 1, 2, 3, or 4,
R1, R2, R3 are independently H or CH3,
wherein at least one AO is PO and at least one further AO is EO.

**4.** The ester F according to any of claims 1 to 3 wherein m1 + m2 + m3 + n1 + n2 + n3 or p1 + p2 + p3 is equal to 3 or 5.

**5.** The ester F according to any of claims 1 to 4 wherein 3 POs are present in total.

**6.** The ester F according to any of claims 1 to 5 wherein at least one PO is present in each of the 3 alkoxy chains of the glycerol.

**7.** A process for preparing an ester F according to any of claims 1 to 6 of alkoxylated glycerol of the formula IIa, IIb or IIc

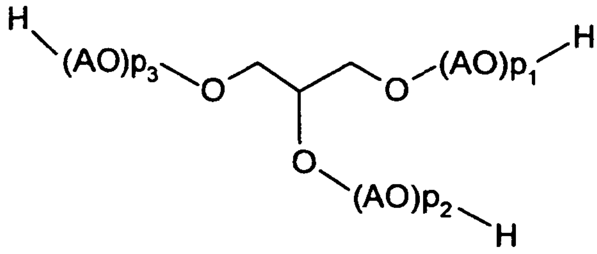

IIa,

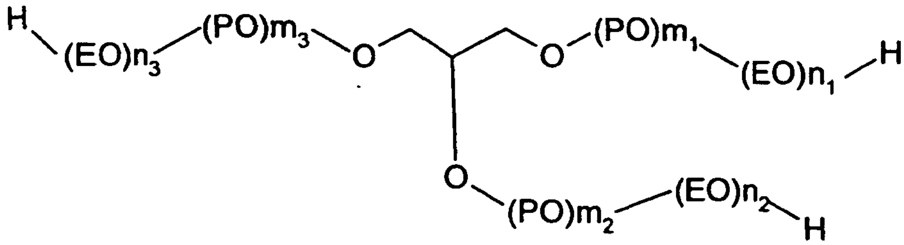

IIb,

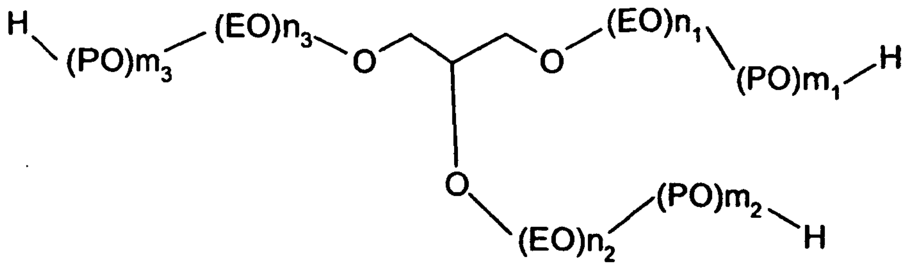

IIc,

where AO, EO, PO, n1, n2, n3, m1, m2, m3, p1, p2 and p3 are each as defined in any of claims 1 to 6, with (meth)acrylic acid, comprising the steps of

a) reacting alkoxylated glycerol with (meth)acrylic acid in the presence of at least one esterification catalyst C and of at least one polymerization inhibitor D and if appropriate also of a water-azeotroping solvent E to form an ester F,
b) if appropriate removing from the reaction mixture some or all of the water formed in a), during and/or after a),
f) if appropriate neutralizing the reaction mixture,
h) when a solvent E was used, if appropriate removing this solvent by distillation, and/or
i) stripping with a gas which is inert under the reaction conditions.

**8.** The process according to claim 7 wherein

- the molar excess of (meth)acrylic acid to alkoxylated glycerol is at least 3.15:1 and
- the if appropriate neutralized (meth)acrylic acid present in the reaction mixture obtained after the last step substantially remains in the reaction mixture.

**9.** The process for preparing an ester F according to any of claims 1 to 6 of alkoxylated glycerol of the formula IIa, IIb or IIc

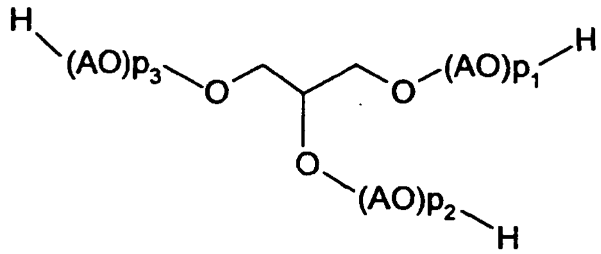

IIa,

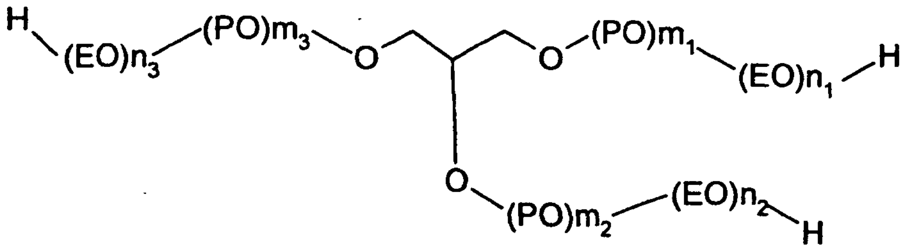

IIb,

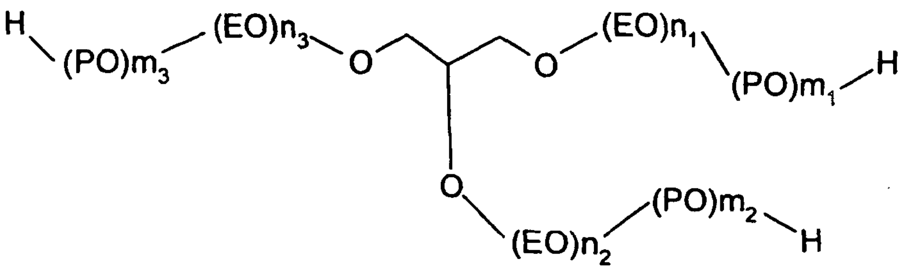

IIc,

where AO is for each AO independently EO or PO,
where EO is O-CH2-CH2-,
PO is at each instance independently O-CH2-CH(CH3)- or O-CH(CH3)-CH2-
p1 + p2 + p3 is 3, 4 or 5,
R1, R2 and R3 are independently H or CH3,
with (meth)acrylic acid, comprising the steps of

a) reacting alkoxylated glycerol with (meth)acrylic acid in the presence of at least one esterification catalyst C and of at least one polymerization inhibitor D and if appropriate also of a water-azeotroping solvent E to form an ester F,
b) if appropriate removing from the reaction mixture some or all of the water formed in a), during and/or after a),
f) if appropriate neutralizing the reaction mixture,
h) when a solvent E was used, if appropriate removing this solvent by distillation, and/or
i) stripping with a gas which is inert under the reaction conditions,

wherein

- the molar excess of (meth)acrylic acid to alkoxylated glycerol is at least 3.15:1 and
- the if appropriate neutralized (meth)acrylic acid present in the reaction mixture obtained after the last step substantially remains in the reaction mixture.

**10.** The process according to any of claims 7 to 9 wherein the (meth)acrylic acid is not more than 75% by weight removed from the reaction mixture obtained after the last step, which reaction mixture comprises ester F.

**11.** The process according to any of claims 7 to 10 wherein the reaction mixture obtained after the last step, which comprises ester F, has a DIN EN 3682 acid number of at least 25 mg of KOH/g.

**12.** The process according to any of claims 7 to 11 wherein the reaction mixture obtained after the last step, which comprises ester F, has a (meth)acrylic acid content of at least 0.5% by weight.

**13.** The process according to any of claims 7 to 12 wherein the molar ratio of (meth)acrylic acid to alkoxylated glycerol in reaction a) is at least 15:1.

**14.** A process for preparing a crosslinked hydrogel, comprising the steps of

k) polymerizing an ester F according to any of claims 1 to 6, or an ester F of the formula Ia

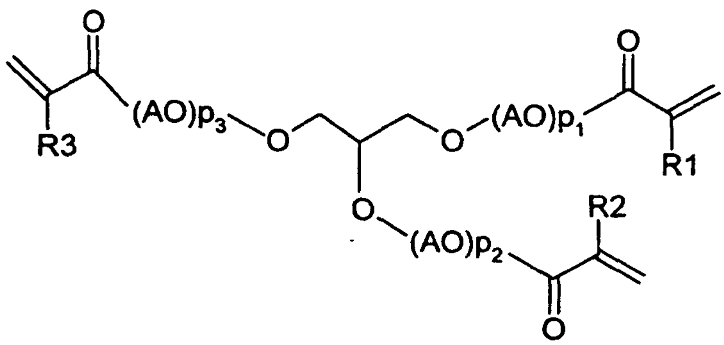

where AO is for each AO independently EO or PO,
where EO is O-CH2-CH2-,
PO is at each instance independently O-CH2-CH(CH3)- or O-CH(CH3)-CH2-
p1 +p2+p3is3,4or5,
R1, R2 and R3 are independently H or CH3, with (meth)acrylic acid, with if appropriate additional monoethylenically unsaturated compounds N and if appropriate also at least one further copolymerizable hydrophilic monomer M in the presence of at least one free-radical initiator K and if appropriate of at least one grafting base L,
l) if appropriate postcrosslinking the reaction mixture obtained from k),
m) drying the reaction mixture obtained from k) or l), and
n) if appropriate grinding and/or sieving the reaction mixture obtained from k), l) or m).

**15.** The process according to claim 14 wherein for the ester F AO is EO.

**16.** A process for preparing a crosslinked hydrogel, comprising steps a) to i) according to any of claims 7 to 15 and additionally

k) polymerizing the reaction mixture from one of stages a) to i) if performed, with if appropriate additional monoethylenically unsaturated compounds N and if appropriate also at least one further copolymerizable hydrophilic monomer M in the presence of at least one free-radical initiator K and if appropriate of at least one grafting base L,
l) if appropriate postcrosslinking the reaction mixture obtained from k),
m) drying the reaction mixture obtained from k) or l), and
n) if appropriate grinding and/or sieving the reaction mixture obtained from k), l) or m).

**17.** A polymer obtainable by a process according to any of claims 14 to 16.

**18.** A crosslinked hydrogel comprising at least one hydrophilic monomer M in copolymerized form and crosslinked with an ester F according to any of claims 1 to 6 or an ester F of formula Ia

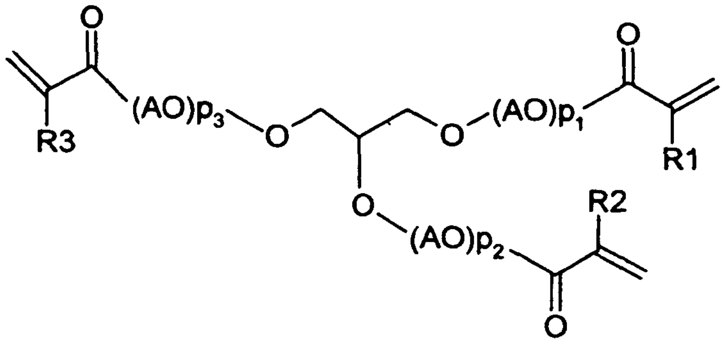

where AO is for each AO independently EO or PO,
where EO is O-CH2-CH2-,
PO is at each instance independently O-CH2-CH(CH3)- or O-CH(CH3)-CH2-
p1 + p2 + p3 is 3, 4 or 5,
R1, R2 and R3 are independently H or CH3.

**19.** A crosslinked hydrogel comprising at least one hydrophilic monomer M in copolymerized form and crosslinked with a reaction mixture comprising ester F and obtainable according to a process of claims 7 to 13.

**20.** The use of a polymer according to any of claims 17 to 19 in hygiene articles, packaging materials and in nonwovens.

**21.** A composition of matter comprising

- from 0.1% to 40% by weight of at least one ester F according to any of claims 1 to 6 or an ester F of formula Ia

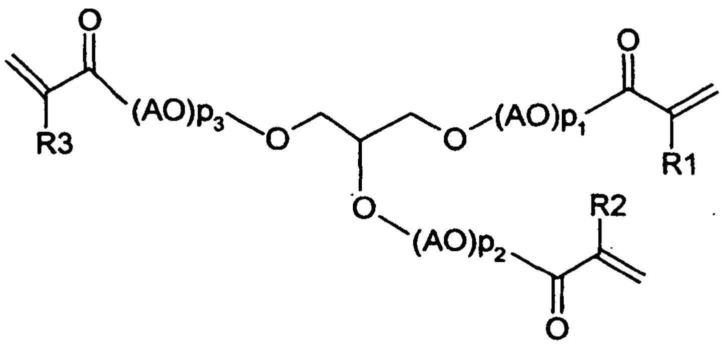

where AO is for each AO independently EO or PO,
where EO is O-CH2-CH2-,
PO is at each instance independently O-CH2-CH(CH3)- or O-CH(CH3)-CH2-
p1 + p2 + p3 is 3,4 or 5,
R1, R2 and R3 are independently H or CH3,
and (meth)acrylic acid,
- 0.5 - 99.9% by weight of at least one hydrophilic monomer M,
- 0 - 10% by weight of at least one esterification catalyst C,
- 0 - 5% by weight of at least one polymerization inhibitor D, and
- 0 - 10% by weight of a solvent E,

with the proviso that the sum total is always 100% by weight.

**22.** A composition of matter according to claim 21, further comprising

- a diluent G ad 100% by weight.

**23.** A crosslinked hydrogel obtainable from a composition of matter according to claim 21 or 22 and additionally

l) if appropriate y postcrosslinking the reaction mixture obtained,
m) drying the reaction mixture obtained directly or from l), and
n) if appropriate grinding and/or sieving the reaction mixture obtained directly or from l) or m).

**24.** The use of a reaction mixture obtainable according to any of claims 7 to 13 or of a composition of matter according to claim 21 or 22

- as a free-radical crosslinker of water-absorbing hydrogels,
- as a starting material for preparing polymer dispersions,
- as a starting material for preparing polyacrylates,
- as a paint raw material, or
- as a cement additive.

**25.** A crosslinked hydrogel having a saponification index of less than 10, preferably less than 8 and especially less than 5.

**26.** The crosslinked hydrogel according to any of claims 17, 18, 19 or 23 having a saponification index of less than 11, preferably less than 10, more preferably less than 8 and especially less than 5.

**27.** The crosslinked hydrogel according to any of claims 17, 18, 19, 23, 25 or 26 having a residual crosslinker content of less than 10 ppm, preferably less than 8 ppm and more preferably less than 5 ppm.

**28.** The use of an ester F according to any of claims 1 to 6 or an ester of formula Ia

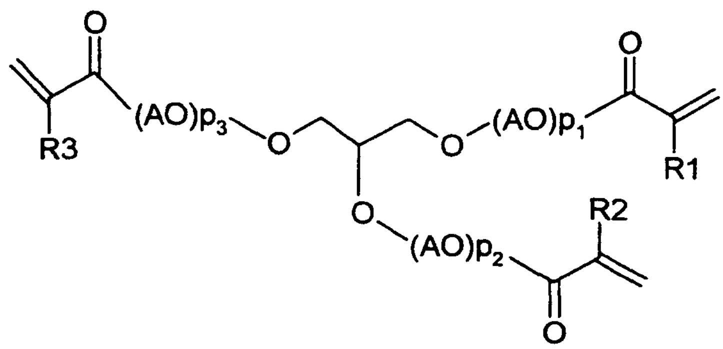

where AO is for each AO independently EO or PO,
where EO is O-CH2-CH2-,
PO is at each instance independently O-CH2-CH(CH3)- or O-CH(CH3)-CH2-
p1 + p2 + p3 is 3, 4 or 5,
R1, R2 and R3 are independently H or CH3,
for preparing hydrogel-forming polymers capable of absorbing aqueous fluids.

## Revendications

**1.** Esters F de la formule Ia :

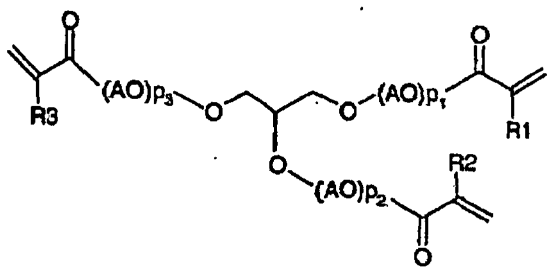

dans laquelle AO représente, pour chaque AO indépendamment, EO ou PO, où
EO représente O-CH2-CH2-,
PO représente indépendamment l'un de l'autre O-CH2-CH(CH3)- ou O-CH(CH3)-CH2-,
p1 + p2 + p3 vaut 3, 4 ou 5,
R1, R2, R3 représentent indépendamment l'un de l'autre H ou CH3,
au moins un AO représentant PO et au moins un autre AO EO.

2. Esters F de la formule Ib :

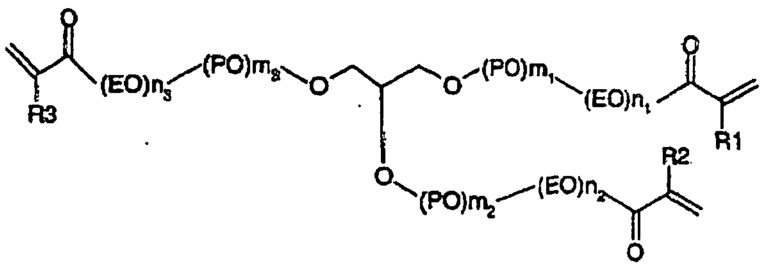

dans laquelle EO représente O-CH2-CH2-,
PO représente indépendamment l'un de l'autre O-CH2-CH(CH3)- ou O-CH(CH3)-CH2-,
m1 + m2 + m3 + n1 + n2 + n3 vaut 3, 4 ou 5,
m1 + m2 + m3 vaut 1, 2, 3 ou 4,
R1, R2, R3 représentent indépendamment l'un de l'autre H ou CH3,
au moins un AO représentant PO et au moins un autre AE EO.

3. Esters de la formule Ic :

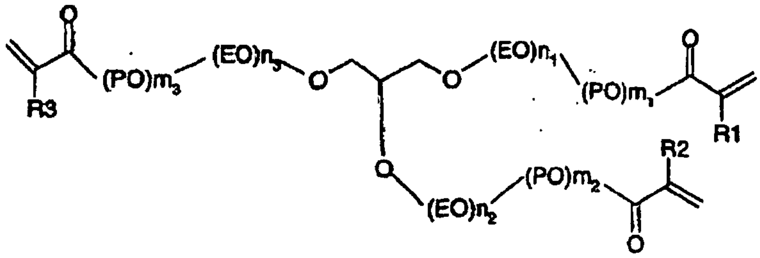

dans laquelle EO représente O-CH2-CH2-,
PO représente indépendamment l'un de l'autre O-CH2-CH(CH3)- ou 0-CH(CH3)-CH2-,
m1 + m2 + m3 + n1 + n2 + n3 vaut 3, 4 ou 5,

m1 + m2 + m3 vaut 1, 2, 3 ou 4,
R1, R2, R3 représentent indépendamment l'un de l'autre H ou CH3,
au moins un AO représentant PO et au moins un autre AE EO.

**4.** Esters F suivant l'une des revendications 1 à 3, où m1 + m2 + m3 + n1 + n2 + n3 ou p1 + p2 + p3 est égal à 3 ou 5.

**5.** Esters F suivant l'une des revendications 1 à 4, dans lesquels il y a au total 3 PO.

**6.** Esters F suivant l'une des revendications 1 à 5, dans lesquels il y a au moins un PO dans chacune des trois chaînes alcoxy de la glycérine.

**7.** Procédé de préparation d'un ester F suivant l'une des revendications 1 à 6 d'une glycérine alcoxylée des formules IIa, IIb ou IIc :

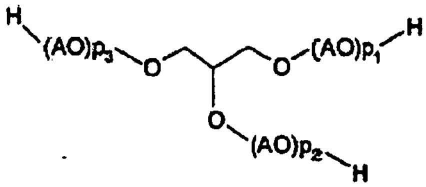
IIa,

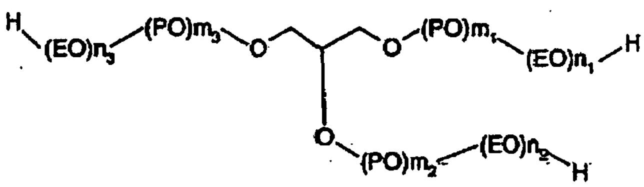
IIb,

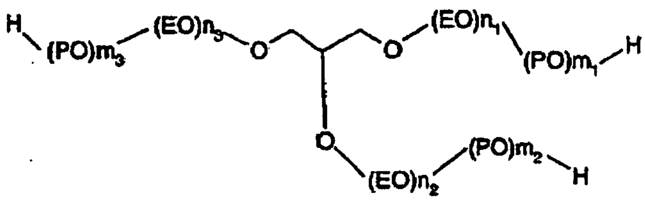
IIc,

dans lesquelles AO, EO, PO, n1, n2, n3, m1, m2, m3, p1, p2, p3 ont la signification donnée dans une des revendications 1 à 6,
avec de l'acide (méth)acrylique, comprenant les étapes

a) de réaction de glycérine alcoxylée avec de l'acide (méth)acrylique en présence d'au moins un catalyseur d'estérification C et d'au moins un inhibiteur de polymérisation D ainsi qu'éventuellement d'un solvant E formant un azéotrope avec l'eau, avec formation d'un ester F,
b) éventuellement d'élimination d'au moins une partie de l'eau formée dans a) hors du mélange réactionnel, b) pouvant avoir lieu pendant et/ou après a),
f) éventuellement de neutralisation du mélange réactionnel,
h) dans le cas où on a mis en oeuvre un solvant E, d'élimination éventuelle de ce solvant par distillation et/ou
i) de stripage à l'aide d'un gaz inerte dans les conditions réactionnelles.

**8.** Procédé suivant la revendication 7, **caractérisé en ce que**

- l'excédant molaire d'acide (méth)acrylique par rapport à la glycérine alcoxylée est d'au moins 3,15/1, et
- l'acide (méth)acrylique éventuellement neutralisé, contenu dans le mélange réactionnel obtenu après la dernière étape, subsiste essentiellement dans le mélange réactionnel.

**9.** Procédé de préparation d'un ester F suivant l'une des revendications 1 à 6 d'une glycérine alcoxylée des formules IIa, IIb ou IIc :

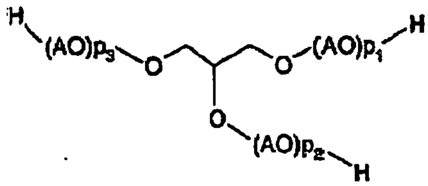

IIa,

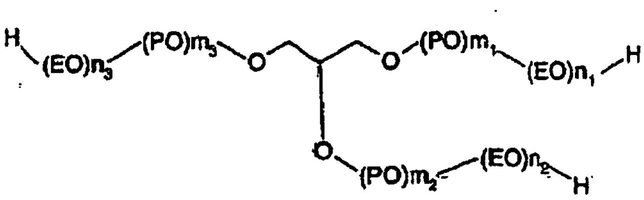

IIb,

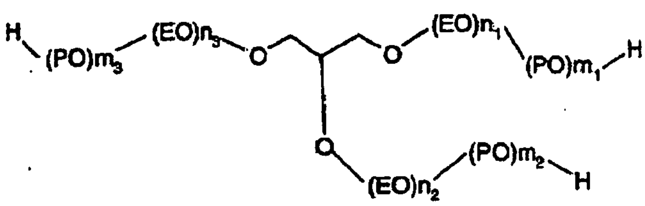

IIc,

dans lesquelles AO représente, pour chaque AO indépendamment, EO ou PO,
EO représente O-CH2-CH2-,
PO représente indépendamment
O-CH2-CH(CH3)- ou O-CH(CH3)-CH2-,
p1 + p2 + p3 vaut 3, 4 ou 5,
R1, R2, R3 représentent indépendamment H ou CH3,
avec de l'acide (méth)acrylique, comportant les étapes

a) de réaction de glycérine alcoxylée avec de l'acide (méth)acrylique en présence d'au moins un catalyseur d'estérification C et d'au moins un inhibiteur de polymérisation D ainsi qu'éventuellement d'un solvant E formant un azéotrope avec l'eau, avec formation d'un ester F,
b) éventuellement d'élimination d'au moins une partie de l'eau formée dans a) à partir du mélange réactionnel, b) pouvant avoir lieu pendant et/ou après a),
f) éventuellement de neutralisation du mélange réactionnel,
h) dans le cas où on a mis en oeuvre un solvant E, d'élimination éventuelle de ce solvant par distillation et/ou
i) de stripage à l'aide d'un gaz inerte dans les conditions réactionnelles,

**caractérisé en ce que**

- l'excédant molaire d'acide (méth)acrylique par rapport à la glycérine alcoxylée est d'au moins 3,15/1, et
- l'acide (méth)acrylique éventuellement neutralisé, contenu dans le mélange réactionnel obtenu après la dernière étape, subsiste essentiellement dans le mélange réactionnel.

**10.** Procédé suivant l'une des revendications 7 à 9, **caractérisé en ce que** l'acide (méth)acrylique est séparé pour pas plus de 75 % en poids du mélange réactionnel contenant l'ester F et obtenu après la dernière étape.

**11.** Procédé suivant l'une des revendications 7 à 10, **caractérisé en ce que** le mélange réactionnel contenant l'ester F et obtenu après la dernière étape présente un indice d'acide selon la norme DIN EN 3682 d'au moins 25 mg de KOH/g.

**12.** Procédé suivant l’une des revendications 7 à 11, **caractérisé en ce que** le mélange réactionnel contenant l’ester F et obtenu après la dernière étape présente une teneur en acide (méth)-acrylique d’au moins 0,5 % en poids.

**13.** Procédé suivant l’une des revendications 7 à 12, **caractérisé en ce que**, dans la réaction a), le rapport molaire entre l’acide (méth)acrylique et la glycérine alcoxylée est d’au moins 15/1.

**14.** Procédé de préparation d’un hydrogel réticulé, comprenant les étapes

k) de polymérisation d’un ester F selon l’une des revendications 1 à 6 ou d’un ester F de la formule Ia :

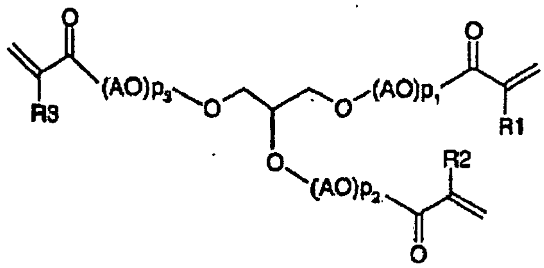

dans laquelle AO représente, pour chaque AO indépendamment, EO ou PO,
EO représente O-CH2-CH2-,
PO représente indépendamment l’un de l’autre O-CH2-CH(CH3)- ou
O-CH(CH3)-CH2-,
p1 + p2 + p3 vaut 3, 4 ou 5,
R1, R2, R3 représentent indépendamment l’un de l’autre H ou CH3,
avec de l’acide (méth)acrylique, avec éventuellement des composés N monoéthyléniquement insaturés additionnels, ainsi qu’éventuellement avec au moins un autre monomère hydrophile M copolymérisable, en présence d’au moins un initiateur radicalaire K et éventuellement d’au moins une base de greffage L,
1) éventuellement de postréticulation du mélange réactionnel obtenu dans k),
m) de séchage du mélange réactionnel obtenu dans k) ou 1), et
n) éventuellement de broyage et/ou de tamisage du mélange réactionnel obtenu dans k), 1) ou m).

**15.** Procédé suivant la revendication 14, dans lequel, dans l’ester F, AO représente EO.

**16.** Procédé de préparation d’un hydrogel réticulé, comprenant les étapes a) à i) suivant l’une des revendications 7 à 15 et, en supplément,

k) une polymérisation du mélange réactionnel d’une des étapes a) à i), dans la mesure où elles ont eu lieu, avec des composés monoéthyléniquement insaturés éventuellement additionnels N, ainsi qu’éventuellement au moins un autre monomère hydrophile M copolymérisable, en présence d’au moins un initiateur radicalaire K et éventuellement d’au moins une base de greffage L,
1) éventuellement une postréticulation du mélange réactionnel obtenu dans k),
m) un séchage du mélange réactionnel obtenu dans k) ou 1), et
n) éventuellement un broyage et/ou tamisage du mélange réactionnel obtenu dans k), 1) ou m).

**17.** Polymère que l’on peut obtenir selon un procédé suivant l’une des revendications 14 à 16.

**18.** Hydrogel réticulé, contenant au moins un monomère hydrophile M sous une forme copolymérisée, qui est réticulé avec un ester F suivant l’une des revendications 1 à 6 ou un ester F de la formule Ia :

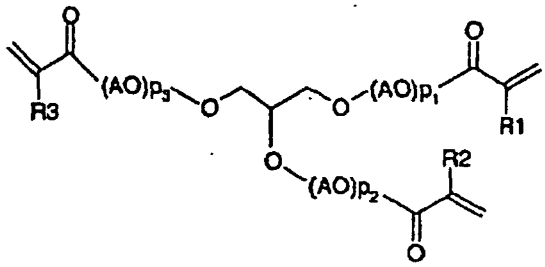

dans laquelle AO représente, pour chaque AO indépendamment, EO ou PO,
EO représente O-CH2-CH2-,
PO représente indépendamment l'un de l'autre O-CH2-CH(CH3)- ou
O-CH(CH3)-CH2-,
p1 + p2 + p3 vaut 3, 4 ou 5,
R1, R2, R3 représentent indépendamment l'un de l'autre H ou CH3.

**19.** Hydrogel réticulé contenant au moins un monomère hydrophile M sous forme copolymérisée, qui est réticulé avec un mélange réactionnel contenant un ester F, tel qu'il peut être obtenu selon un procédé suivant les revendications 7 à 13.

**20.** Utilisation d'un polymère suivant l'une des revendications 17 à 19, dans des articles d'hygiène, des matières d'emballage et des non-tissés.

**21.** Mélange de matières, contenant

- 0,1 à 40 % en poids d'au moins un ester F suivant l'une des revendications 1 à 6 ou d'un ester F de la formule Ia :

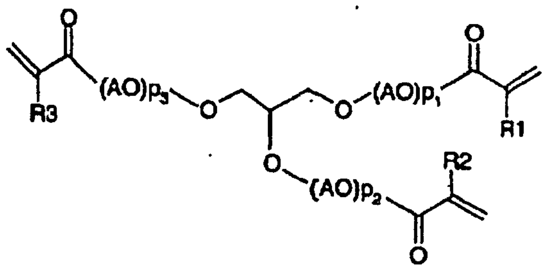

dans laquelle AO représente, pour chaque AO indépendamment, EO ou PO,
EO représente O-CH2-CH2-,
PO représente indépendamment l'un de l'autre O-CH2-CH(CH3)- ou
O-CH(CH3)-CH2-,
p1 + p2 + p3 vaut 3, 4 ou 5,
R1, R2, R3 représentent indépendamment l'un de l'autre H ou CH3,
et d'acide (méth) acrylique,

- 0,5-99,9 % en poids d'au moins un monomère hydrophile M,
- 0-10 % en poids d'au moins un catalyseur d'estérification C,
- 0-5 % en poids d'au moins un inhibiteur de polymérisation D, et
- 0-10 % en poids d'un solvant E,

à la condition que la somme soit toujours égale à 100 % en poids.

**22.** Mélange de matières suivant la revendication 21, contenant en supplément

- un diluant G jusqu'à 100 % en poids.

**23.** Hydrogel réticulé, que l'on peut obtenir à partir d'un mélange de matières suivant la revendication 21 ou 22, et en supplément par

l) éventuellement postréticulation du mélange réactionnel obtenu,
m) séchage du mélange réactionnel directement obtenu ou obtenu dans 1), et
n) éventuellement broyage et/ou tamisage du mélange réactionnel obtenu directement ou dans 1) ou m).

**24.** Utilisation d'un mélange réactionnel que l'on peut obtenir suivant l'une des revendications 7 à 13 ou d'un mélange de matières suivant la revendication 21 ou 22

- comme agent de réticulation radicalaire d'hydrogels absorbant l'eau,
- comme matière de départ pour la préparation de dispersions de polymère,
- comme matière de départ pour la préparation de polyacrylates,
- comme matière première de vernis, ou
- comme additif de ciment.

**25.** Hydrogel réticulé présentant un indice de saponification inférieur à 10, de préférence inférieur à 8, en particulier inférieur à 5.

**26.** Hydrogel réticulé suivant l'une des revendications 17, 18, 19 ou 23 présentant un indice de saponification inférieur à 11, de préférence inférieur à 10, particulièrement avantageusement inférieur à 8, en particulier inférieur à 5.

**27.** Hydrogel réticulé suivant l'une des revendications 17, 18, 19, 23, 25 ou 26, présentant une teneur en agent de réticulation résiduaire inférieure à 10 ppm, de préférence inférieure à 8 ppm, particulièrement avantageusement inférieure à 5 ppm.

**28.** Utilisation d'un ester F suivant l'une des revendications 1 à 6 ou d'un ester de la formule Ia :

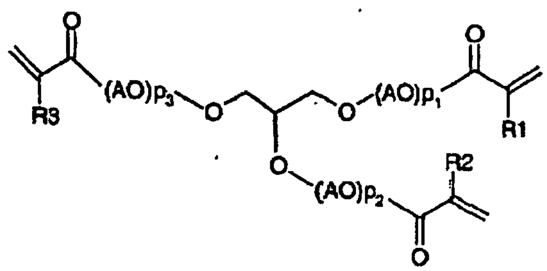

dans laquelle AO représente, pour chaque AO indépendamment, EO ou PO,
EO représente O-CH2-CH2-,
PO représente indépendamment l'un de l'autre O-CH2-CH(CH3)- ou O-CH(CH3)-CH2-,
p1 + p2 + p3 vaut 3, 4 ou 5,
R1, R2, R3 représentent indépendamment l'un de l'autre H ou CH3,
pour la préparation de polymères formant des hydrogels absorbant des liquides aqueux.